# EUROPEAN PATENT APPLICATION

(11) **EP 4 772 504 A1**
(43) Date of publication of application: **08.07.2026**
(21) Application number: 23960567.8
(22) Date of filing: 08.12.2023
(51) Int. Cl.: C07D 401/14, C07D 401/12, C07D 471/04, C07B 59/00, C07F 1/08, C07F 5/00, C07F 5/06, C07F 9/94, C07F 13/00, A61K 51/04, A61K 103/00, A61P 35/00

(54) **RADIOLABELED FAPI COMPLEX, PREPARATION METHOD THEREFOR, AND USE THEREOF**

(71) Applicant: Beijing Normal University, Beijing 100088 (CN)
(72) Inventor: ZHU, Lin, Beijing 100088 (CN); LUO, Yang, Beijing 100088 (CN); JIN, Wenbin, Beijing 100088 (CN); HONG, Haiyan, Beijing 100088 (CN); YAN, Li, Beijing 100088 (CN); HUANG, Yanran, Beijing 100088 (CN); KUNG, Hank F, Philadelphia Pennsylvania 19104 (US)
(74) Representative: JD&P Patent Attorneys Joanna Dargiewicz & Partners
(86) International application number: PCT/CN2023/137303
(87) International publication number: WO 2025/118255

(57) **Abstract**

The invention relates to a radiolabeled FAPI complex, and a preparation method therefor and a use thereof, and belongs to the technical field of radiopharmaceutical chemistry and nuclear medicine theranostics; and a general structural formula of FAPI capable of realizing radionuclide labeling is as shown below:

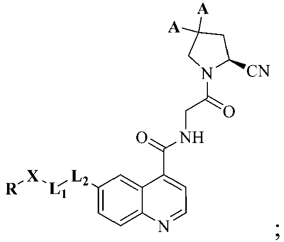

R is a nitroimidazole group or -COOH; X is a chelating group or a chelating structure chelated with a radionuclide; L₁ and L₂ are linking groups; and A is an adjusting atom of an FAP-targeting group, and all the A atoms are H atoms or F atoms. The radionuclide-labeled precursor of the invention has a simple synthetic method, is suitable for labeling with various nuclides, and achieves convenient labeling, and a label has high radiochemical purity and good stability; and the radiolabeled FAPI complex shows high affinity and specificity for FAP in cell experiments, and high tumor uptake and target-to-non-target ratio in tumor-bearing mice, thereby being a potential FAP-targeting radioactive theranostic drug.

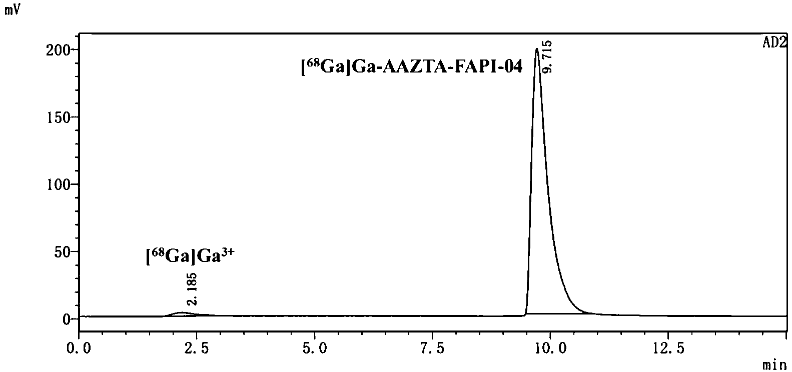

## Description

### TECHNICAL FIELD

The present invention relates to a novel radionuclide-labeled complex, and a preparation method therefor and a use thereof, and particularly to a radiolabeled FAPI complex, and a preparation method therefor and a use thereof, and belongs to the technical field of radiopharmaceutical chemistry and nuclear medicine theranostics.

### BACKGROUND OF THE PRESENT INVENTION

Fibroblast activation protein-α (FAP) is not expressed or expressed at a low level in healthy adult tissues, but the expression thereof is increased in non-malignant diseases involving tissue remodeling such as wound healing, liver cirrhosis, arthritis, atherosclerosis and fibrosis, and related to disease progression. More importantly, the FAP is highly expressed in stromal cancer-associated fibroblasts in more than 90% of epithelial cancers. Therefore, the FAP is a potential target for tumor detection and therapy.

Radionuclide-labeled quinoline-based small-molecule inhibitors targeting the fibroblast activation protein (FAPI) have shown promising results in the diagnosis of tumors and other diseases, among which FAPI-04 is the most representative, with favorable affinity and specificity toward the FAP. Ga-68-labeled [⁶⁸Ga]Ga-FAPI-04 is rapidly cleared via the kidneys in vivo, and displays a favorable tumor-to-background ratio in PET imaging. Meanwhile, the FAPI-04 may also be labeled with a therapeutic radionuclide such as Lu-177. However, for radiotherapeutic drugs, the FAPI-04 has a relatively low tumor uptake, and in particular, tumor retention time of the FAPI-04 still needs to be increased. Therefore, further increasing tumor uptake and retention time of a drug while reducing a non-target tissue uptake represents the current direction and objective of FAPI drug development.

Since 2018, a non-pharmacophore moiety in a structure of the FAPI has been optimized in a lot of studies, but these results have showed only limited enhancement in the tumor uptake and retention time of the FAPI, thereby failing to fully meet the requirements for clinical theranostic applications. In 2021, the inventors developed a Ga-68-labeled FAPI-based radioactive molecular probe (Patent No.: ZL202111437982.7). Such drugs have high affinity and specificity for the FAP, and show a high tumor uptake in tumor-bearing mice. However, these drugs are metabolized through hepatic and intestinal systems, showing high uptake and slow clearance in the liver, gallbladder and intestines, thereby leaving a room for further optimization.

All currently reported FAPIs have the defects of insufficient target uptake and rapid elution, resulting in low target-to-non-target ratios, which are unfavorable for disease diagnostic imaging and therapy.

Therefore, a fibroblast activation protein inhibitor (FAPI), and a preparation method therefor and a use thereof are provided, wherein the FAPI has a simple synthetic method, is suitable for labeling with a variety of radionuclides, and achieves convenient labeling, and a label has high radiochemical purity and favorable stability; and a radionuclide-labeled FAPI complex shows high affinity and specificity toward the FAP in cell experiments, indicating high tumor uptake and target-to-non-target ratio in tumor-bearing mice. A novel FAPI capable of being rapidly and efficiently taken up and retained for a long period in target tissues in vivo and being rapidly cleared from non-target organs will improve uptake and retention time of the drug at a target site, and reduce a damage to blood and non-target organs, there by being a promising FAP-targeted radioactive theranostic drug.

### SUMMARY OF THE PRESENT INVENTION

One objective of the present invention is to develop a novel radiolabeled FAPI complex, in which, by effective binding of an FAPI group to various chelating groups and linking groups, a tumor uptake of the drug is increased and tumor retention time of the drug is prolonged, thereby overcoming the defects of low uptake and short tumor retention time of the radiolabeled FAPI compound reported in current literatures, which are unfavorable for clinical tumor theranostics; the novel radiolabeled FAPI complex has a simple synthetic method, is suitable for labeling with various radionuclides, and achieves convenient labeling, and a label has high radiochemical purity and good stability; and the radiolabeled FAPI complex shows high affinity and specificity for FAP in cell experiments, and a very high tumor uptake, very long tumor retention time and an excellent target-to-non-target ratio in tumor-bearing mice, thereby being a potential novel FAP-targeting radioactive theranostic drug.

The above objective of the present invention is achieved by the following technical solution.

A radiolabeled FAPI complex is provided, wherein the radiolabeled FAPI complex has a general structural formula as follows: 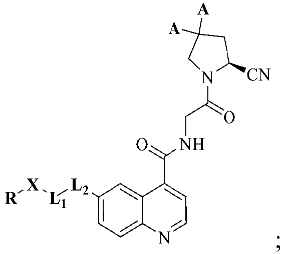 wherein, R is a nitroimidazole group or -COOH, and the nitroimidazole group is selected from any one of the following groups:

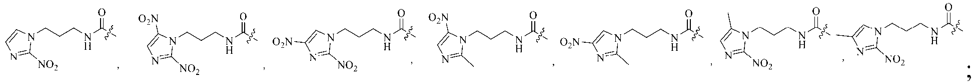

X is a chelating group or a chelating structure chelated with a radionuclide, and selected from any one of the following groups: 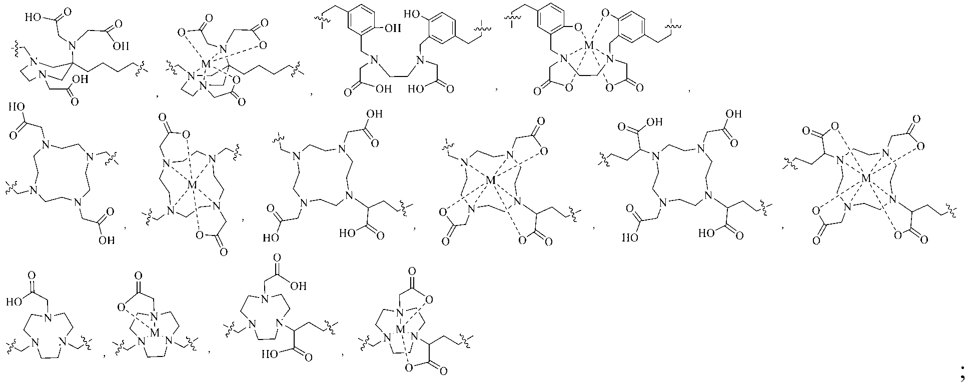
wherein, M includes, but is not limited to, ⁶⁷Ga³⁺, ⁶⁸Ga³⁺, [Al¹⁸F]²⁺, ⁶⁴Cu²⁺, ⁶⁷Cu²⁺, ¹¹¹In³⁺, ¹⁷⁷Lu³⁺, ⁸⁶Y³⁺, ⁹⁰Y³⁺, ⁴⁴Sc³⁺, ⁴⁷Sc³⁺, ²²⁵Ac³⁺, ²¹²Pb²⁺, ²⁰³Pb²⁺, ²¹³Bi³⁺, ²¹²Bi³⁺, and the like;
L₁ and L₂ are linking groups, wherein the L₁ is selected from any one of the following groups: 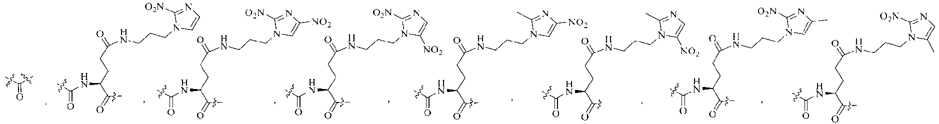 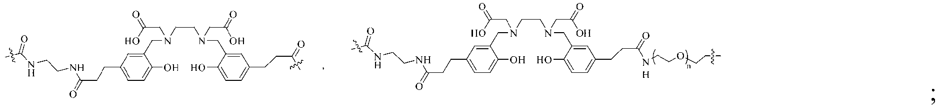
wherein, n is an integer from 0 to 6;
the L₂ is selected from any one of the following groups:

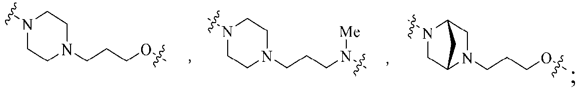

A is an adjusting atom of an FAP-targeting group, and all the A atoms are H atoms or F atoms.

Preferably, a specific structure is:

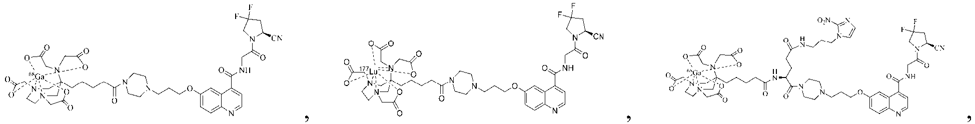

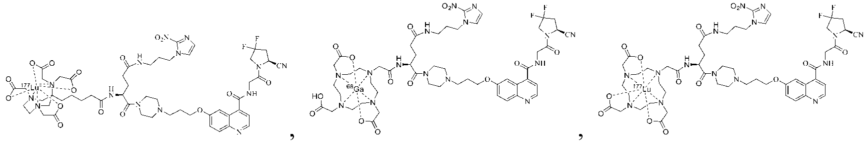

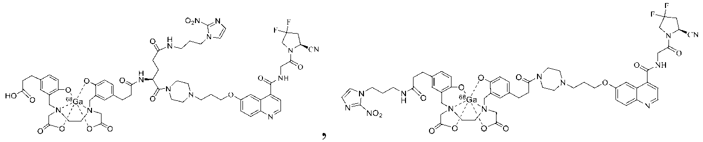

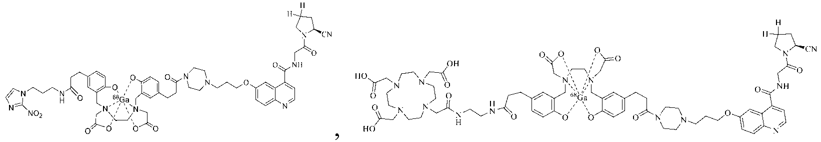

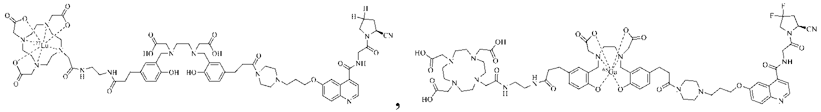

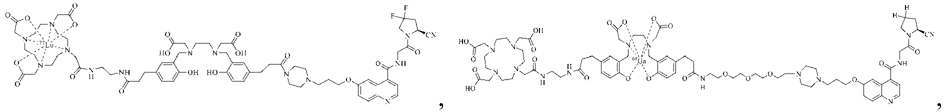

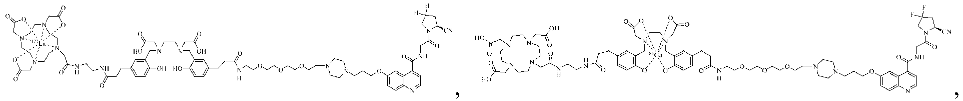

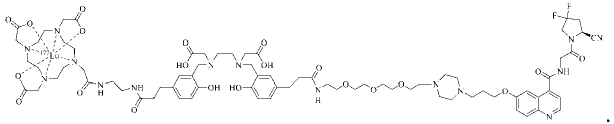

Another objective of the present invention is to provide a preparation method for the radiolabeled FAPI complex above.

The above objective of the present invention is achieved by the following technical solution.

A preparation method for a radiolabeled FAPI complex is provided, which includes steps as follows:
dissolving nitroimidazole and anhydrous potassium carbonate in ultra-dry N,N-dimethylformamide, stirring at room temperature, and adding tert-butyl N-(3-bromopropyl)carbamate, and after the reaction is completed, subjecting the reaction mixture to suction filtration and extraction, drying the organic phase, and purifying to obtain a yellow-green oily compound, and deprotecting the yellow-green oily compound with trifluoroacetic acid to obtain a white solid intermediate 1;
dissolving (*S*)-4-((((9H-fluoren-9-yl)methoxy)carbonyl)amino)-5-(tert-butoxy)-5-oxopentanoicacidin ultra-dry *N,N*-dimethylformamide, adding 2-(7-azabenzotriazol-1-yl)-*N*,*N*,*N'*,*N'*-tetramethy-luronium hexafluorophosphate and *N,N*'-diisopropylethylamine under ice bath and stirring, and adding the white solid intermediate 1 obtained in the step (1), after the reaction is completed, extracting the reaction mixture, drying the organic phase, and purifying to obtain a pale yellow solid, deprotecting the pale yellow solid with trifluoroacetic acid to obtain a yellow oily substance, dissolving the yellow oily substance in *N,N-*dimethylformamide, adding 2-(7-azabenzotriazol-1-yl)-*N*,*N*,*N'*,*N'*-tetramethyluronium hexafluorophosphate and *N,N'*-diisopropylethylamine under ice bath and stirring, adding FAPI deprotected with trifluoroacetic acid to remove a Boc group (specifically including (*S*)-6-[3-(4-Boc-1-piperazinyl)-propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S,*4*S*)-tert-butyl 5-(3-((4-((2-((*S*)-2-cyano-4,4-difluoropyrrolidin-1-yl)-2-oxoethyl)-carbamoyl)quinolin-6-yl)oxy)propyl)-2,5-diazabicyclo[2.2.1]heptane-2-carboxylate, (*S*)-tert-butyl 4-(3-((4-((2-(2-cyano-4,4-difluoropyrrolidin-1-yl)-2-oxoethyl)carbamoyl)-quinolin-6-yl)(methyl)amino)propyl)piperazine-1-carboxylate), and after the reaction is completed, extracting the reaction mixture, drying the organic phase, and purifying to obtain a pale yellow oily substance, and deprotecting the pale yellow oily substance with diethylamine to obtain a pale yellow oily intermediate 2 (NI-FAPI);
dissolving FAPI (specifically including (*S*)-6-[3-(4-Boc-1-piperazinyl)-propoxy]-*N*[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)propyl)-2,5-diazabicyclo[2.2.1]heptane-2-carboxylate, (*S*)-tert-butyl 4-(3-((4-((2-(2-cyano-4,4-difluoropyrrolidin-1-yl)-2-oxoethyl)carbamoyl)quino-lin-6-yl)(methyl)amino)propyl)piperazine-1-carboxylate) in trifluoroacetic acid, stirring at room temperature, removing the solvent by rotary evaporation to obtain a yellow oily substance, dissolving the yellow oily substance in anhydrous acetonitrile, adding anhydrous potassium carbonate, stirring at room temperature, adding a PEG chain (BocNH(CH₂CH₂O)ₙCH₂CH₂Br) under ice bath, refluxing the mixture to react overnight, subjecting the reaction mixture to suction filtration, removing the solvent by evaporation, and purifying to obtain a pale yellow oiy intermediate 3 (PEGₙ-FAPI);
dissolving a chelating agent in ultra-dry *N,N*-dimethylformamide, adding 2-(7-azabenzotriazol-1-yl)-*N*,*N*,*N'*,*N'*-tetramethyluronium hexafluorophosphate and *N,N*'-diisopropylethylamine under ice bath and stirring, and adding *N*-Cbz-1,2-ethanediamine, and after the reaction is completed, extracting the reaction mixture with ethyl acetate and saturated brine, drying the organic phase with anhydrous sodium sulfate, purifying by silica gel column chromatography to obtain a yellow solid, and deprotecting the yellow solid with hydrogen to obtain a white solid substance; and dissolving 3,3'-(((2,2,13,13-tetramethyl-4,11-dioxo-3,12-dioxa-6,9-diazatetradecane-6,9-diyl)bis(methylene ))bis(4-hydroxy-3,1-phenylene))dipropanoic acid (HBED-CC(*t*Bu)₂) in ultra-dry *N,N*-dimethylformamide, adding 2-(7-azabenzotriazol-1-yl)-*N*,*N*,*N'*,*N'*-tetramethyluronium hexafluorophosphate and *N,N'*-diisopropylethylamine under ice bath and stirring, and adding the white solid substance, and after the reaction is completed, extracting the reaction mixture, drying the organic phase, and purifying to obtain a colorless solid intermediate 4 (Chelator-HBED-CC(*t*Bu)₂);
dissolving a chelating agent in ultra-dry *N,N*-dimethylformamide, adding 2-(7-azabenzotriazol-1-yl)-*N*,*N*,*N'*,*N'*-tetramethyluronium hexafluorophosphate and *N,N'*-diisopropylethylamine under ice bath and stirring, and adding the intermediate 2 prepared in the step (2) or FAPI deprotected with trifluoroacetic acid to remove a Boc group (specifically including (*S*)-6-[3-(4-Boc-1-piperazinyl)propoxy]-N-[2-(2-cyano-4,4-difluoro-1-pyrrolidinyl)-2-oxoethyl]-quinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1*S*,4*S*)-tert-butyl 5-(3-((4-((2-((*S*)-2-cyano-4,4-di-fluoropyrrolidin-1-yl)-2-oxoethyl)carbamoyl)quinolin-6-yl)oxy)propyl)-2,5-diazabicyclo[2.2.1]-heptane-2-carboxylate and (*S*)-tert-butyl 4-(3-((4-((2-(2-cyano-4,4-difluoropyroli-din-1-yl)-2-oxoethyl)carbamoyl)quinolin-6-yl)(methyl)amino)propyl)piperazine-1-carboxylate); and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain a Chelator-NI-FAPI or Chelator-FAPI labeling precursor;
dissolving a chelating agent in ultra-dry *N,N*-dimethylformamide, adding 2-(7-azabenzotriazol-1-yl)-*N*,*N*,*N'*,*N'*-tetramethyluronium hexafluorophosphate and N,N'-diisopropylethylamine under ice bath and stirring, and adding the intermediate 1 prepared in the step (1) to react at room temperature overnight, after the reaction is completed, extracting the reaction mixture, drying the organic phase, purifying to obtain a yellow oily substance, dissolving the yellow oily substance in ultra-dry *N,N*-dimethylformamide, adding 2-(7-azabenzotriazol-1-yl)-*N*,*N*,*N'*,*N'*-tetramethyluronium hexafluorophosphate and *N,N*'-diisopropylethylamine under ice bath and stirring, and adding FAPI deprotected with trifluoroacetic acid to remove a Boc group (specifically including (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)p ropyl)-2,5-diazabicyclo[2.2.1]heptane-2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl) amino)propyl)piperazine-1-carboxylate) to react at room temperature overnight, and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain an NI-Chelator-FAPI labeling precursor;
dissolving the intermediate 4 prepared in the step (4) in *N,N*-dimethylformamide, adding 2-(7-azabenzotriazol-1-yl)-*N*,*N*,*N'*,*N'*-tetramethyluronium hexafluorophosphate and *N,N*'-diisopropylethylamine under ice bath and stirring, and adding FAPI deprotected with trifluoroacetic acid to remove a Boc group (specifically including (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)p ropyl)-2,5-diazabicyclo[2.2.1]heptane-2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl) amino)propyl)piperazine-1-carboxylate) or the intermediate 3 prepared in the step (3), and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain a Chelator-HBED-CC-(PEG)ₙ-FAPI labeling precursor; and
dissolving the labeling precursors obtained in the steps (5), (6) and (7) in dimethyl sulfoxide, adding a sodium acetate buffer solution and a radionuclide-containing solution, and heating to obtain a corresponding radiolabeled product.

Preferably, in the step (1), the nitroimidazole is 2-nitroimidazole, and an amount of the nitroimidazole added is 1 eq.; an amount of the anhydrous potassium carbonate added is 3-5 eq.; an amount of the ultra-dry *N,N*-dimethylformamide added is 10-20 mL; and an amount of the tert-butyl *N*-(3-bromopropyl)carbamate added is 1-2 eq.

Preferably, in the step (1), the reaction mixture is subjected to suction filtration through celite, the filtrate is extracted with ethyl acetate and saturated brine, and the organic phase is dried with anhydrous sodium sulfate, and purified by silica gel column chromatography.

Preferably, in the step (2), an amount of the (*S*)-4-((((9H-fluo-ren-9-yl)methoxy)carbonyl)amino)-5-(tert-butoxy)-5-oxopentanoic acid is 1 eq.; an amount of the ultra-dry *N,N*-dimethylformamide added is 10-20 mL; and an amount of the 2-(7-azabenzotriazol-1-yl)-*N*,*N*,*N'*,*N'*-tetramethyluronium hexafluorophosphate and the *N,N*'-diisopropylethylamine added is 1-2 eq.

Preferably, in the step (2), an amount of the yellow oily substance added is 1-1.5 1 eq.; an amount of the ultra-dry *N,N*-dimethylformamide added is 5-10 mL; an amount of the 2-(7-azabenzotriazol-1-yl)-*N*,*N*,*N'*,*N'*-tetramethyluronium hexafluorophosphate and the *N,N'*-diisopropylethylamine added is 1.3-1.5 eq.; and an amount of the FAPI deprotected with trifluoroacetic acid to remove the Boc group (specifically including (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidi-nyl)-2-oxoethyl]quinoline-4-carboxamide, (1*S,*4*S*)-tert-butyl 5-(3-((4-((2-((*S*)-2-cyano-4,4-di-fluoropyrrolidin-1-yl)-2-oxoethyl)carbamoyl)quinolin-6-yl)oxy)propyl)-2,5-diazabicyclo[2.2.1]h eptane-2-carboxylate, (S)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added is 1 eq.

Preferably, in the step (2), the reaction mixture is extracted with ethyl acetate and saturated brine, and the organic phase is dried with anhydrous sodium sulfate and then purified by silica gel column chromatography.

Preferably, in the step (3), the mixture is refluxed to react at 80°C; and the reaction mixture is subjected to suction filtration through celite to remove potassium carbonate, the solvent is removed by rotary evaporation under reduced pressure from the filtrate, and the residue is purified by the flash chromatograph.

Preferably, in the step (3), an amount of the FAPI (specifically including (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)--2-oxoethyl]quinoline-4-carboxamide, (1*S*,4*S*)-tert-butyl 5-(3-((4-((2-((*S*)-2-cyano-4,4-difluoro-pyrrolidin-1-yl)-2-oxoethyl)carbamoyl)quinolin-6-yl)oxy)propyl)-2,5-diazabicyclo[2.2.1]heptane -2-carboxylate, (*S*)-tert-butyl 4-(3-((4-((2-(2-cyano-4,4-difluoropyrrolidin-1-yl)-2-oxoethyl)-carbamoyl)quinolin-6-yl)(methyl)amino)propyl)piperazine-1-carboxylate) added is 1 eq.; and an amount of the trifluoroacetic acid added is 3-5 mL.

Preferably, in the step (3), an amount of the yellow oily substance added is 1 eq.; an amount of the anhydrous potassium carbonate added is 4-5 eq.; and the PEG chain is tert-butyl (2-(2-(2-(2-bromoethoxy)ethoxy)ethoxy)ethyl)carbamate, an amount of which is 1.2-1.5 eq.

Preferably, in the step (4), the chelating agent includes 5-(6-(bis(2-(tert-butoxy)-2-oxoethyl)amino)-1,4-bis(2-(tert-butoxy)-2-oxoethyl)-1,4-diazepan-6-yl)pentanoic acid (AAZTA-(*t*Bu)₄), 2-(4,7,10-tris(2-(tert-butoxy)-2-oxoethyl)-1,4,7,10-tetraazacyclododecan-1-yl)acetic acid (DOTA(*t*Bu)₃), 5-(tert-butoxy)-5-oxo-4-(4,7,10-tris(2-(tert-butoxy)-2-oxoethyl)-1,4,7,10-tetra-azacyclododecan-1-yl)pentanoic acid (DOTAGA(*t*Bu)₄), 4-(4,10-bis(2-(tert-butoxy)-2-oxoethyl)-7-(1,5-di(tert-butoxy)-1,5-dioxopentan-2-yl)-1,4,7,10-tetraazacyclododecan-1-yl)-5-(tert-b utoxy)-5-oxopentanoic acid (DOTA(GA)₂ (*t*Bu)₅), 2-(4,7-bis(2-(tert-butoxy)-2-oxoethyl)-1,4,7-triazacyclopentan-1-yl)acetic acid (NOTA(*t*Bu)₂) or 4-(4,7-bis(2-(tert-butoxy)--2-oxoethyl)-1,4,7-triazolan-1-yl)-5-(tert-butoxy)-5-oxopentanoic acid (NODAGA(*t*Bu)₃).

Preferably, in the step (4), an amount of the chelating agent added is 1 eq.; an amount of the ultra-dry *N,N*-dimethylformamide added is 15-20 mL; an amount of the 2-(7-azabenzotriazol-1-yl)-*N*,*N*,*N'*,*N'*-tetramethyluronium hexafluorophosphate added is 1 eq.; an amount of the *N,N*'-diisopropylethylamine added is 3 eq.; and an amount of the *N*-Cbz-1,2-ethanediamine added is 1.2-1.5 eq.

Preferably, in the step (4), the reaction mixture is extracted with ethyl acetate and saturated brine, and the organic phase is dried with anhydrous sodium sulfate, and purified by silica gel column chromatography to obtain the yellow solid; an amount of the HBED-CC(*t*Bu)₂ added is 1 eq.; an amount of the ultra-dry N,N-dimethylformamide added is 10-20 mL; an amount of the 2-(7-azabenzotriazol-1-yl)-*N*,*N*,*N'*,*N'*-tetramethyluronium hexafluorophosphate added is 1 eq.; and an amount of the *N,N*'-diisopropylethylamine added is 3 eq.

Preferably, in the step (4), the white solid substance is added, and after the reaction is completed, the reaction mixture is extracted with ethyl acetate and saturated brine, the organic phase is dried with anhydrous sodium sulfate, and purified by the flash chromatograph to obtain the colorless solid intermediate 4 (Chelator-HBED-CC(*t*Bu)₂).

Preferably, in the step (5), the chelating agent is 5-(6-(bis(2-(tert-butoxy)-2-oxoethyl)-amino)-1,4-bis(2-(tert-butoxy)-2-oxoethyl)-1,4-diazepan-6-yl)pentanoic acid (AAZTA(*t*Bu)₄), 2-(4,7,10-tris(2-(tert-butoxy)-2-oxoethyl)-1,4,7,10-tetraazacyclododecan-1-yl)acetic acid (DOTA(*t*Bu)₃), 3,3'-(((2,2,13,13-tetramethyl-4,11-dioxo-3,12-dioxa-6,9-diazatetradecane-6,9-di-yl)bis(methylene))bis(4-hydroxy-3,1-phenylene))dipropanoic acid (HBED-CC(*t*Bu)₂), 5-(tert-butoxy)-5-oxo-4-(4,7,10-tris(2-(tert-butoxy)-2-oxoethyl)-1,4,7,10-tetraazacyclododecan-1 -yl)pentanoic acid (DOTAGA(*t*Bu)₄), 4-(4,10-bis(2-(tert-butoxy)-2-oxoethyl)-7-(1,5-di-(tert-butoxy)-1,5-dioxopentan-2-yl)-1,4,7,10-tetraazacyclododecan-1-yl)-5-(tert-butoxy)-5-oxope ntanoic acid (DOTA(GA)₂ (*t*Bu)₃), 2-(4,7-bis(2-(tert-butoxy)-2-oxoethyl)-1,4,7-triaza-cyclopentan-1-yl)acetic acid (NOTA(*t*Bu)₂) or 4-(4,7-bis(2-(tert-butoxy)-2-oxoethyl)-1,4,7-tri-azolan-1-yl)-5-(tert-butoxy)-5-oxopentanoic acid (NODAGA(*t*Bu)₃).

Preferably, in the step (5), an amount of the chelating agent added is 1-1.2 eq.; an amount of the ultra-dry *N,N*-dimethylformamide added is 2-5 mL; an amount of the 2-(7-azabenzotriazol-1-yl)-*N*,*N*,*N'*,*N'*-tetramethyluronium hexafluorophosphate added is 1-1.5 eq.; an amount of the *N,N*'-diisopropylethylamine added is 1.5-3 eq.; an amount of the intermediate 2 or the FAPI deprotected with trifluoroacetic acid to remove the Boc group (specifically including (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)--2-oxoethyl]quinoline-4-carboxamide, (1*S*,4*S*)-tert-butyl 5-(3-((4-((2-((*S*)-2-cyano-4,4-difluoro-pyrrolidin-1-yl)-2-oxoethyl)carbamoyl)quinolin-6-yl)oxy)propyl)-2,5-diazabicyclo[2.2.1]heptane -2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added is 1 eq.; and after the reaction is completed, the reaction mixture is extracted with ethyl acetate and saturated brine, and the organic phase is dried with anhydrous sodium sulfate, and purified by the flash chromatograph.

Preferably, in the step (6), the chelating agent is 3,3'-(((2,2,13,13-tetramethyl-4,11-dioxo-3,12-dioxa-6,9-diazatetradecane-6,9-diyl)bis(methylene))bis(4-hydroxy-3,1-phenylene))dipropanoic acid (HBED-CC(*t*Bu)₂) or 4,4'-(4,10-bis(2-(tert-butoxy)-2-oxoethyl)-1,4,7,10-tetraazacyclododecane-1,7-diyl)bis(5-(tert-butoxy)-5-oxopentanoic acid) (DOTA(GA)₂(*t*Bu)₄).

Preferably, in the step (6), an amount of the chelating agent added is 1 eq.; an amount of the ultra-dry *N,N*-dimethylformamide added is 2-10 mL; an amount of the 2-(7-azabenzotriazol-1-yl)-*N*,*N*,*N'*,*N*'-tetramethyluronium hexafluorophosphate added is 1 eq.; an amount of the *N,N'*-diisopropylethylamine added is 3-5 eq.; and an amount of the intermediate 1 added is 1 eq.

Preferably, in the step (6), after the reaction is completed, the reaction mixture is extracted with ethyl acetate and saturated brine, and the organic phase is dried with anhydrous sodium sulfate, and purified by the flash chromatograph.

Preferably, in the step (6), amounts of the pale yellow oily substance, the 2-(7-azabenzotriazol-1-yl)-*N*,*N*,*N'*,*N'*-tetramethyluronium hexafluorophosphate, the *N,N*'-diisopropylethylamine and the FAPI deprotected with trifluoroacetic acid to remove the Boc group (specifically including (*S*)-6-[3-(4-Boc-1-piperazinyl)propoxy]-N-[2-(2-cyano-4,4-di-fluoro-1-pyrrolidinyl)-2-oxoethyl]quinoline-4-carboxamide, (*S*)-6-[3-(4-Boc-1-piperazinyl)pro-poxy]-*N*-[2-(2-cyanopyrrolidinyl)-2-oxoethyl]quinoline-4-carboxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)p ropyl)-2,5-diazabicyclo[2.2.1]heptane-2-carboxylate and (*S*)-tert-butyl 4-(3-((4-((2-(2-cyano--4,4-difluoropyrrolidin-1-yl)-2-oxoethyl)carbamoyl)quinolin-6-yl)(methyl)amino)propyl)piperaz ine-1-carboxylate) added are all 1 eq.; and an amount of the ultra-dry *N,N*-dimethylformamide added is 7-10 mL

Preferably, in the step (6), after the reaction is completed, the reaction mixture is extracted with ethyl acetate and saturated brine, and the organic phase is dried with anhydrous sodium sulfate, and purified by the flash chromatograph.

Preferably, in the step (7), an amount of the intermediate 4 added is 1 eq.; an amount of the 2-(7-azabenzotriazol-1-yl)-*N*,*N*,*N'*,*N'*-tetramethyluronium hexafluorophosphate added is 1-1.5 eq.; an amount of the *N,N'*-diisopropylethylamine added is 4-5 eq.; and an amount of the intermediate 3 or the FAPI deprotected with trifluoroacetic acid to remove the Boc group (specifically including (S)-6-[3-(4-Boc-1-piperazinyl)propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]N-[2-(2-cyanopyrrolidinyl)-2-oxoethyl]quinoline-4-carboxamide, (1*S*,4*S*)-tert-butyl 5-(3-((4-((2-((*S*)-2-cyano4,4-difluoropyrrolidin-1-yl)-2-oxoethyl)carbamoyl)quinolin-6-yl)oxy)propyl)-2,5-diazab icyclo[2.2.1]heptane-2-carboxylate and (*S*)-tert-butyl 4-(3-((4-((2-(2-cyano-4,4-difluoropyro-lidin-1-yl)-2-oxoethyl)carbamoyl)quinolin-6-yl)(methyl)amino)propyl)piperazine-1-carboxylate) added is 1-1.5 eq.

Preferably, in the step (7), ; after the reaction is completed, the reaction mixture is extracted with ethyl acetate and saturated brine, and the organic phase is dried with anhydrous sodium sulfate, and purified by the flash chromatograph.

Preferably, in the step (8), the radionuclide-containing solution is [⁶⁸Ga]GaCl₃, [¹⁷⁷Lu]LuCl₃ or [¹⁸F]AlF.

Another objective of the present invention is to provide a use of the radiolabeled FAPI complex above.

The above objective of the present invention is achieved by the following technical solution.

A use of the radiolabeled FAPI complex in manufacture of a targeted radioactive theranostic drug is provided.

### Beneficial effects:

In the novel radiolabeled FAPI complex of the present invention, by effective binding of an FAPI group to various chelating groups and linking groups, tumor uptake and retention time of the drug are increased. The novel radiolabeled FAPI complex has a simple synthetic method, is suitable for labeling with various nuclides, and achieves convenient labeling, and a label has high radiochemical purity and good stability; and the radionuclide-labeled compound shows high affinity and specificity for FAP in cell experiments, and high tumor uptake and target-to-non-target ratio in tumor-bearing mice, thereby being a potential FAP-targeting radioactive theranostic drug.

The present invention is further described hereinafter with reference to the drawings and specific embodiments, which do not imply any limitation on the scope of protection of the present invention.

### BRIEF DESCRIPTION OF THE DRAWINGS

FIG. 1 shows a radioactive HPLC chromatogram of a labeling reaction solution of [⁶⁸Ga]Ga-AAZTA-FAPI-04 prepared in Example 1 of the present invention.
FIG. 2 shows a radioactive HPLC chromatogram of a labeling reaction solution of [¹⁷⁷Lu]Lu-AAZTA-FAPI-04 prepared in Example 2 of the present invention.
FIG. 3 shows a radioactive HPLC chromatogram of a labeling reaction solution of [⁶⁸Ga]Ga-AAZTA-NI-FAPI-04 prepared in Example 3 of the present invention.
FIG. 4 shows a radioactive HPLC chromatogram of a labeling reaction solution of [¹⁷⁷Lu]Lu-AAZTA-NI-FAPI-04 prepared in Example 4 of the present invention.
FIG. 5 shows a radioactive HPLC chromatogram of a labeling reaction solution of [⁶⁸Ga]Ga-DOTA-NI-FAPI-04 prepared in Example 5 of the present invention.
FIG. 6 shows a radioactive HPLC chromatogram of a labeling reaction solution of [¹⁷⁷Lu]Lu-DOTA-NI-FAPI-04 prepared in Example 6 of the present invention.
FIG. 7 shows a radioactive HPLC chromatogram of a labeling reaction solution of [⁶⁸Ga]Ga-HBED-CC-NI-FAPI-04 prepared in Example 7 of the present invention.
FIG. 8 shows a radioactive HPLC chromatogram of a labeling reaction solution of [⁶⁸Ga]Ga-NI-HBED-CC-FAPI-04 prepared in Example 8 of the present invention.
FIG. 9 shows a radioactive HPLC chromatogram of a labeling reaction solution of [⁶⁸Ga]Ga-NI-HBED-CC-FAPI-02 prepared in Example 9 of the present invention.
FIG. 10 shows a radioactive HPLC chromatogram of a labeling reaction solution of DOTA-[⁶⁸Ga]Ga-HBED-CC-FAPI-02 prepared in Example 10 of the present invention.
FIG. 11 shows a radioactive HPLC chromatogram of a labeling reaction solution of [¹⁷⁷Lu]Lu-DOTA-HBED-CC-FAPI-02 prepared in Example 11 of the present invention.
FIG. 12 shows a radioactive HPLC chromatogram of a labeling reaction solution of DOTA-[⁶⁸Ga]Ga-HBED-CC-FAPI-04 prepared in Example 12 of the present invention.
FIG. 13 shows a radioactive HPLC chromatogram of a labeling reaction solution of [¹⁷⁷Lu]Lu-DOTA-HBED-CC-FAPI-04 prepared in Example 13 of the present invention.
FIG. 14 shows a radioactive HPLC chromatogram of a labeling reaction solution of DOTA-[⁶⁸Ga]Ga-HBED-CC-PEG₃-FAPI-02 prepared in Example 14 of the present invention.
FIG. 15 shows a radioactive HPLC chromatogram of a labeling reaction solution of [¹⁷⁷Lu]Lu-DOTA-HBED-CC-PEG₃-FAPI-02 prepared in Example 15 of the present invention.
FIG. 16 shows a radioactive HPLC chromatogram of a labeling reaction solution of DOTA-[⁶⁸Ga]Ga-HBED-CC-PEG₃-FAPI-04 prepared in Example 16 of the present invention.
FIG. 17 shows a radioactive HPLC chromatogram of a labeling reaction solution of [¹⁷⁷Lu]Lu-DOTA-HBED-CC-PEG₃-FAPI-04 prepared in Example 17 of the present invention.
FIG. 18 shows uptake-time curves of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in HT1080-FAP cells in vitro in Application Example 1 of the present invention.
FIG. 19 shows uptake and blocking results at 60 minutes of DOTA-[⁶⁸Ga]Ga-HBED-CC-FAPI-02 (**A**), DOTA-[⁶⁸Ga] Ga-HBED-CC-FAPI-04 (**B**), DOTA-[⁶⁸Ga]Ga-HBED-CC-PEG₃-FAPI-02 (**C**), DOTA-[⁶⁸Ga]Ga-HBED-CC-PEG₃-FAPI-04 (**D**) and ⁶⁸Ga]Ga-DOTA-FAPI-04 (**E**) in HT1080-FAP cells in vitro in Application Example 1 of the present invention.
FIG. 20 shows uptake-time curves of [¹⁷⁷Lu]Lu-DOTA-HBED-CC-FAPI-02, [¹⁷⁷Lu]Lu-DOTA-HBED-CC-FAPI-04, [¹⁷⁷Lu]Lu-DOTA-HBED-CC-PEG;-FAPI-02, [¹⁷⁷Lu]Lu-DOTA-HBED-CC-PEG₃-FAPI-04 and [¹⁷⁷Lu]Lu-DOTA-FAPI-04 in HT1080-FAP cells in vitro in Application Example 2 of the present invention.
FIG. 21 shows uptake and blocking results at 60 minutes of [¹⁷⁷Lu]Lu-DOTA-HBED-CC-FAPI-02 (**A**), [¹⁷⁷Lu]Lu-DOTA-HBED-CC-FAPI-04 (**B**), [¹⁷⁷Lu]Lu-DOTA-HBED-CC-PEG₃-FAPI-02 (**C**), [¹⁷⁷Lu]Lu-DOTA-HBED-CC-PEG₃-FAPI-04 (**D**) and [¹⁷⁷Lu]Lu-DOTA-FAPI-04 (**E**) in HT1080-FAP cells in vitro in Application Example 2 of the present invention.
FIG. 22 shows uptake-time curves of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 in HT1080-FAP cells in vitro in Application Example 3 of the present invention.
FIG. 23 shows uptake and blocking results at 60 minutes of [¹⁷⁷Lu]Lu-AAZTA-FAPI-04 (**F**), [¹⁷⁷Lu]Lu-AAZTA-NI-FAPI-04 (**G**), [¹⁷⁷Lu]Lu-DOTA-NI-FAPI-04 (**H**) and [¹⁷⁷Lu]Lu-DOTA-FAPI-04 (**E**) in HT1080-FAP cells in vitro in Application Example 3 of the present invention.
FIG. 24 shows uptake-time curves of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 in HT1080-FAP cells in vitro in Application Example 4 of the present invention.
FIG. 25 shows uptake and blocking results at 60 minutes of[⁶⁸Ga]Ga-HBED-CC-NI-FAPI-04 (**A**), [⁶⁸Ga]Ga-NI-HBED-CC-FAPI-04 (**B**), [⁶⁸Ga]Ga-NI-HBED-CC-FAPI-02 (**C**) and [⁶⁸Ga]Ga-DOTA-FAPI-04 (**D**) in HT1080-FAP cells in vitro in Application Example 4 of the present invention.
FIG. 26 shows PET/CT imaging results in U87MG tumor-bearing mice of [⁶⁸Ga]Ga-AAZTA-FAPI-04, [⁶⁸Ga]Ga-AAZTA-NI-FAPI-04, [⁶⁸Ga]Ga-DOTA-NI-FAPI-04, DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in Application Example 5 of the present invention (T: tumor; G: gallbladder).

### DETAILED DESCRIPTION OF PREFERRED EMBODIMENTS

Unless otherwise specified, the reagents and raw materials used in the preparation and detection methods described in the following examples and comparative examples are all commercially available products, and the devices used are all conventional devices; and the concentrations and ratios are all based on weight and molar units.

### Example 1: preparation of [⁶⁸Ga]Ga-AAZTA-FAPI-04

### Step 1: synthesis of AAZTA-FAPI-04

### A synthetic route was as follows:

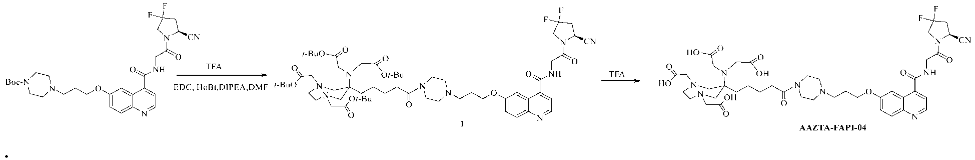

The synthetic route specifically included the following steps.

### (1) Synthesis of compound 1

In a 10 mL round-bottom flask, (S)-6-[3-(4-Boc-1-piperazinyl)propoxy]-N-[2-(2-cyano-4,4-difluoro-1-pyrrolidinyl)-2-oxoethyl]quinoline-4-carboxamide (CAS: 2374782-82-6, 50 mg, 0.09 mmol) was dissolved in 2 mL of trifluoroacetic acid, stirred at room temperature for 30 minutes, subjected to rotary evaporation under reduced pressure to remove the solvent, added with 3 mL of ultra-dry *N,N-*dimethylformamide, added with 1-ethyl-(3-dimethylaminopropyl)carbodiimide hydrochloride (25.60 mg, 0.13 mmol), 1-hydroxybenzotriazole (18.00 mg, 0.13 mmol) and *N*,*N*-diisopropylethylamine (39.00 mg, 0.30 mmol) under ice bath, and then added with 5-(6-(bis(2-(tert-butoxy)-2-oxoethyl)amino)-1,4-bis(2-tert-butoxy-2-oxoethyl)-1,4-diazacycloheptan-6-yl)pentanoic acid (AAZTA(*t*Bu)₄, 57.70 mg, 0.09 mmol) dissolved in 2 mL of ultra-dry *N,N-*dimethylformamide. The reaction mixture was allowed to react at room temperature overnight, and then the reaction mixture was diluted with ethyl acetate, washed with ethyl acetate (20 mL × 3) and saturated brine (10 mL × 1). The organic phase was dried with anhydrous sodium sulfate and filtered, and the filtrate was subjected to rotary evaporation under reduced pressure to remove the solvent. The residue was purified by silica gel column chromatography (dichloromethane/methanol/ammonia water, v/v/v = 20/1/0.1) to obtain the orange-yellow compound **1** (64.70 mg, 0.06 mmol), with a yield of 67%.

### Structural confirmation of compound 1:

HRMS C₅₈H₈₈N₉O₁₂F₂ [M+H]⁺ calcd for 1140.6526, found 1140.6520.

¹H NMR (400 MHz, CDCl₃) δ 8.82 (d, *J* = 4.4 Hz, 1H), 8.04 (d, *J* = 9.2 Hz, 1H), 7.50 (d, *J* = 4.3 Hz, 1H), 7.40 (d, *J =* 8.9 Hz, 1H), 4.54 - 3.89 (m, 6H), 3.61 (s, 4H), 3.22 (s, 4H), 2.99 (d, *J* = 13.9 Hz, 2H), 2.80 (dd, *J =* 18.4, 9.1 Hz, 4H), 2.64 (d, *J =* 14.1 Hz, 5H), 2.33 (s,2H), 1.65 - 1.53 (m, 6H), 1.44 (d, *J =* 3.8 Hz, 31H), 1.25 (s,14H), 0.88 (t, *J =* 6.8 Hz, 4H).

### (2) Synthesis of compound AAZTA-FAPI-04

In a 10 mL round-bottom flask, the compound **1** (64.70 mg, 0.06 mmol) was dissolved in 2 mL of trifluoroacetic acid, stirred at room temperature for 30 minutes, and subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-12 min, 5%-25% B; 12-14 min, 25%-5% B; 14-15 min, 5% B, flow rate: 4 mL/min; ultraviolet: 280 nm). The target fraction was lyophilized by a lyophilizer to obtain the white solid compound AAZTA-FAPI-04, with a purity of >97% as determined by LC-MS.

### Structural confirmation of AAZTA-FAPI-04:

HRMS C₄₂H₅₆N₉O₁₂F₂ [M+H]⁺ calcd for 916.4011, found 916.4016.

### Step 2: Ga-68 labeling of AAZTA-FAPI-04

A Ga-68 labeling route was as follows:

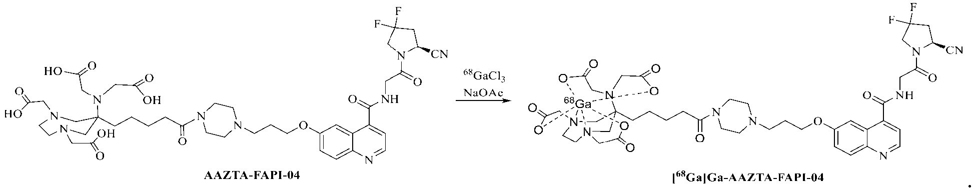

A labeling method was as follows.

The AAZTA-FAPI-04 obtained in the step 1 was prepared into a precursor solution with a concentration of 1 µg/µL from dimethyl sulfoxide, and 9 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 50°C for 10 minutes, and a radiochemical purity was determined by high-performance liquid chromatography equipped with a radioactivity detector, so as to obtain [⁶⁸Ga]Ga-AAZTA-FAPI-04 with a radiochemical purity greater than 95%.

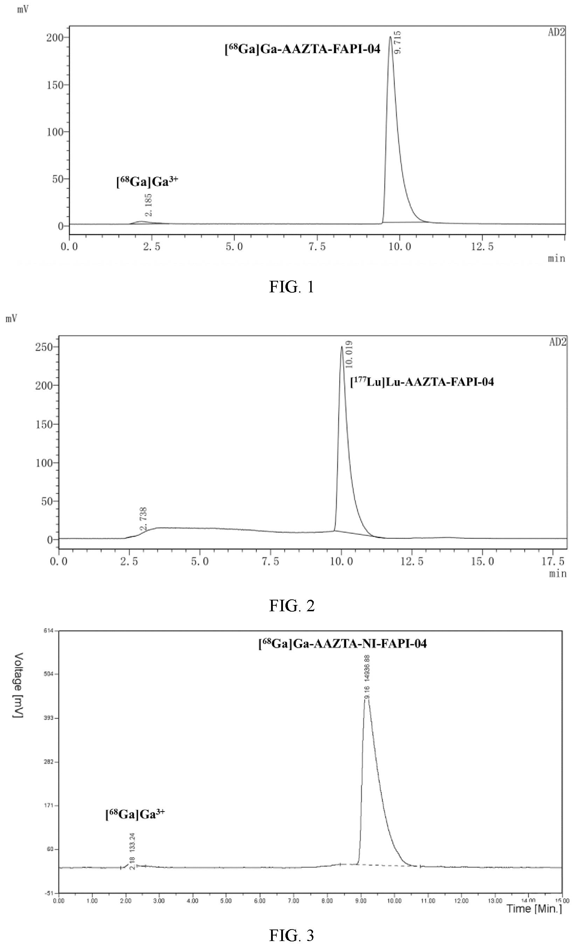

FIG. 1 shows a radioactive HPLC chromatogram of a labeling reaction solution of [⁶⁸Ga]Ga-AAZTA-FAPI-04 prepared in Example 1 of the present invention, and the chromatogram indicates that the radiochemical purity of [⁶⁸Ga]Ga-AAZTA-FAPI-04 is greater than 95%.

### Example 2: preparation of [¹⁷⁷Lu]Lu-AAZTA-FAPI-04

### Step 1: synthesis of AAZTA-FAPI-04

A synthetic route and a synthetic method were identical to those in the step 1 of Example 1.

### Step 2: Lu-177 labeling of AAZTA-FAPI-04

A Lu-177 labeling route was as follows:

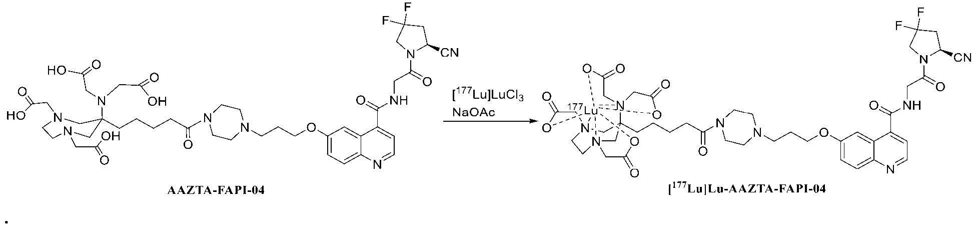

A labeling method was as follows.

The AAZTA-FAPI-04 obtained in the step 1 was prepared into a precursor solution with a concentration of 1 µg/µL from dimethyl sulfoxide, and 18 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 50°C for 20 minutes, and a radiochemical purity was determined by high-performance liquid chromatography equipped with a radioactivity detector, so as to obtain [¹⁷⁷Lu]Lu-AAZTA-FAPI-04 with a radiochemical purity greater than 95%.

FIG. 2 shows a radioactive HPLC chromatogram of a labeling reaction solution of [¹⁷⁷Lu]Lu-AAZTA-FAPI-04 prepared in Example 2 of the present invention, and the chromatogram indicates that the radiochemical purity of [¹⁷⁷Lu]Lu-AAZTA-FAPI-04 is greater than 95%.

### Example 3: preparation of [⁶⁸Ga]Ga-AAZTA-NI-FAPI-04

### Step 1: synthesis of AAZTA-NI-FAPI-04

A synthetic route was as follows:

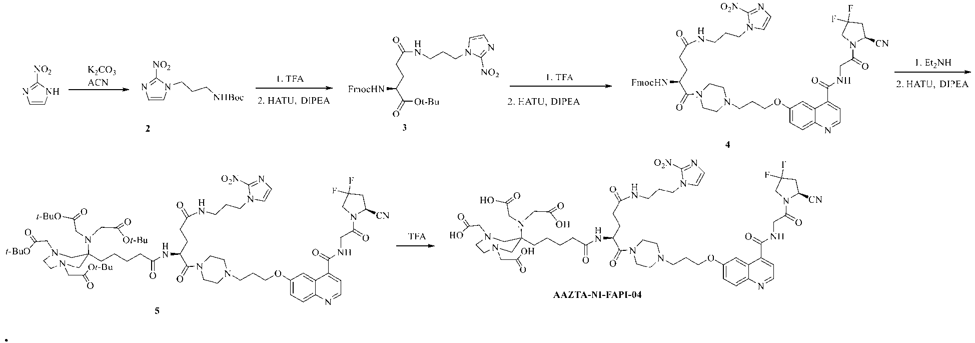

The synthetic route specifically included the following steps.

### (1) Synthesis of compound 2

2-nitroimidazole (1.14 g, 10.09 mmol) was dissolved in 15 mL of ultra-dry N,N-dimethylformamide, added with anhydrous potassium carbonate (4.89 g, 35.38 mmol), and stirred at room temperature for 30 minutes to obtain a reaction mixture. Tert-butyl N-(3-bromopropyl)carbamate (3.57 g, 14.99 mmol) dissolved in ultra-dry N,N-dimethylformamide (10 mL) was added into the above reaction mixture, stirred at room temperature overnight, and filtered through celite, and the filtrate was subjected to rotary evaporation under reduced pressure to remove most of the solvent. The residue was washed with ethyl acetate (20 mL × 3) and saturated brine (10 mL × 2), the organic phase was collected, dried with anhydrous sodium sulfate, and filtered to remove the anhydrous sodium sulfate solid, and the filtrate was subjected to rotary evaporation under reduced pressure to remove the solvent. The residue was purified by silica gel column chromatography (petroleum ether/ethyl acetate, v/v = 1/1) to obtain the yellowish-green oily compound **2** (2.36 g, 8.74 mmol), with a yield of 87%.

### Structural confirmation of compound 2:

HRMS C₁₁H₁₉N₄O₄ [M+H]⁺calcd for 271.1400, found 271.1408;
¹H NMR (600 MHz, CDCl₃) δ 7.27 (s,1H), 7.14 (s,1H), 4.75 (s,1H), 4.46 (t, *J*=7.0 Hz,2H), 3.20 (t, *J*=6.2 Hz,2H), 2.08-2.01 (m,2H), 1.44 (s,9H).

### (2) Synthesis of compound 3

The compound **2** (432 mg, 1.60 mmol) was dissolved in 4 mL of trifluoroacetic acid solution, stirred at room temperature for 30 minutes, and subjected to rotary evaporation under reduced pressure to remove the solvent, so as to obtain a white solid intermediate. (*S*)-4-((((9H-fluoren-9-yl)methoxy)carbonyl)amino )-5-(tert-butoxy)-5-oxopentanoic acid (1.5 g, 3.53 mmol) was dissolved in 10 mL of ultra-dry *N,N-*dimethylformamide, added with 2-(7-azabenzotriazol-1-yl)-*N,N,N,N*'-tetramethyluronium hexafluorophosphate (1.61 g, 4.23 mmol) and *N,N'*-diisopropylethylamine (547 mg, 4.23 mmol) under ice bath, and stirred under ice bath for 25 minutes. The mixture was added with the above white solid intermediate (603 mg, 3.55 mmol), stirred at room temperature overnight, and washed with ethyl acetate (20 mL × 3) and saturated brine (20 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by silica gel column chromatography (dichloromethane/methanol/ammonia water, v/v/v = 25/1/0.1) to obtain the pale yellow solid compound **3** (1.5 g, 2.60 mmol), with a yield of 74%.

### Structural confirmation of compound 3:

HRMS C₃₀H₃₆N₅O₇ [M+H]⁺ calcd for 578.2609, found 578.2609;
¹H NMR (400 MHz, CDCl₃) δ 7.76 (d, *J =* 7.6 Hz,2H), 7.59 (t, *J =* 7.2 Hz,2H), 7.40 (t, *J* = 7.5 Hz,2H), 7.35 - 7.28 (m,2H), 7.27 (s,1H), 7.09 (s,1H), 6.57 (s,1H), 5.65 (d, *J =* 7.2 Hz,1H), 4.41 (dd, *J =* 12.1, 7.0 Hz,4H), 4.21 (t, *J* = 7.0 Hz,2H), 3.42 - 3.32 (m, 1H), 3.31 - 3.21 (m, 1H), 2.35 - 2.18 (m,3H), 2.10 - 1.99 (m,2H), 1.92 - 1.81 (m, 1H), 1.47 (s,9H).

### (2) Synthesis of compound 4

The compound **3** (319.6 mg, 0.55 mmol) was dissolved in 2 mL of dichloromethane, then added with 2 mL of trifluoroacetic acid, and stirred at room temperature for 30 minutes. The reaction was monitored by TLC, and the reaction mixture was subjected to rotary evaporation under reduced pressure to remove the solvent, so as to obtain a yellow oily compound. The yellow oily compound (151.2 mg, 0.29 mmol) was dissolved in 2 mL of ultra-dry *N,N*-dimethylformamide, added with 1-ethyl-(3-dimethylaminopropyl)carbodiimide hydrochloride (74.8 mg, 0.39 mmol), 1-hydroxybenzotriazole (52.7 mg, 0.39 mmol) and *N,N'*-diisopropylethylamine (134.4 mg, 1.04 mmol) under ice bath, and stirred under ice bath for 30 minutes to obtain a reaction mixture. (*S*)-6-[3-(4-Boc-1-piperazinyl)propoxy]-N-[2-(2-cyano-4,4-difluoro-1-pyrrolidinyl)-2-oxoethyl] quinoline-4-carboxamide (CAS: 2374782-82-6, 126.4 mg, 0.26 mmol) deprotected with trifluoroacetic acid and dissolved in 2 mL of ultra-dry *N,N*-dimethylformamide was added into the above reaction mixture. The reaction was carried out at room temperature overnight, and the reaction mixture was washed with ethyl acetate (20 mL × 3) and saturated brine (20 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 15/1/0.1) to obtain the pale yellow oily compound **4** (205.6 mg, 0.21 mmol), with a yield of 81%.

### Structural confirmation of compound 4:

HRMS C₅₀H₅₄N₁₁O₉F₂ [M+H]⁺ calcd for 990.4068, found 990.4076;
¹H NMR (600 MHz, CD₃OD) δ 8.69 (d, *J =* 4.4 Hz,1H), 7.90 (dd, *J =* 16.1, 5.8 Hz,2H), 7.73 (d, *J* = 7.5 Hz,2H), 7.60 (dd, *J =* 11.1, 7.6 Hz,2H), 7.51 (d, *J* = 4.4 Hz,1H), 7.45 (s,1H), 7.40 (dd, *J* = 9.2, 2.6 Hz,1H), 7.32 (t, *J* = 7.4 Hz,2H), 7.25 (ddd, *J* = 10.1, 5.5, 2.1 Hz,2H), 7.03 (s,1H), 5.07 (dd, *J* = 9.3, 2.7 Hz,1H), 4.57 (dd, *J* = 9.7, 3.9 Hz,1H), 4.44 - 4.37 (m,2H), 4.34 (dd, *J =* 10.5, 6.9 Hz,1H), 4.28 - 4.20 (m,4H), 4.20 - 4.12 (m,2H), 4.12 - 4.02 (m,1H), 3.67 - 3.58 (m,2H), 3.58 - 3.49 (m, 1H), 3.27 (dt, *J* = 3.1, 1.5 Hz,3H), 3.15 - 3.07 (m, 1H), 2.85 (ddt, *J =* 14.0, 9.5, 6.9 Hz,1H), 2.73 (t, *J =* 13.6 Hz,1H), 2.61 - 2.44 (m,5H), 2.32 - 2.20 (m,2H), 1.98 (dtd, *J* = 20.6, 13.8, 6.9 Hz,5H), 1.74 (td, *J =* 15.4, 6.0 Hz,1H), 1.33 (dd, *J =* 26.1, 13.4 Hz,1H), 1.29 - 1.21 (m,3H).

### (2) Synthesis of compound 5

The compound **4** (50.5 mg, 0.05 mmol) was dissolved in 2 mL of dichloromethane, added with 1 mL of diethylamine, and stirred at room temperature for 2 hours. The reaction was monitored by TLC, and the reaction mixture was subjected to rotary evaporation under reduced pressure to remove the solvent, so as to obtain a pale yellow oily intermediate. 5-(6-(bis(2-(tert-butoxy)-2-oxoethyl)amino)-1,4-bis(2-tert-butoxy-2-oxoethyl)-1,4-diazacyclohep tan-6-yl)pentanoic acid (AAZTA(*t*Bu)₄, 42 mg, 0.06 mmol) was dissolved in 2 mL of ultra-dry *N,N*-dimethylformamide, added with 2-(7-azabenzotriazol-1-yl)-*N*,*N*,*N'*,*N'*-tetramethyluronium hexafluorophosphate (23 mg, 0.06 mmol) and *N,N'*-diisopropylethylamine (13 mg, 0.1 mmol) under ice bath, and stirred under ice bath for 30 minutes to obtain a reaction mixture. The pale yellow oily intermediate (38.37 mg, 0.05 mmol) dissolved in 2 mL of ultra-dry *N,N-*dimethylformamide was added into the above reaction mixture to react at room temperature overnight, and washed with ethyl acetate (10 mL × 3) and saturated brine (10 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 8/1/0.1) to obtain the yellow oily compound **5** (29 mg, 0.02 mmol), with a yield of 40%.
HRMS C₆₉H₁₀₃N₁₄O₁₆F₂ [M+H]⁺ calcd for 1421.7639, found 1421.7646;
¹H NMR (600 MHz, CD₃OD) δ 8.75 (d, *J =* 4.1 Hz,1H), 7.96 (dd, *J =* 10.7, 5.6 Hz,2H), 7.64 - 7.52 (m,2H), 7.46 (d, *J =* 9.1 Hz,1H), 7.13 (s,1H), 4.50 (d, *J =* 3.3 Hz,2H), 4.36 - 4.21 (m,5H), 4.15 (dt, *J =* 20.1, 10.3 Hz,1H), 3.70 (s,2H), 3.66 - 3.51 (m,5H), 3.36 - 3.24 (m,7H), 3.23 - 3.16 (m,2H), 2.94 (dt, *J =* 23.4, 9.4 Hz,2H), 2.82 (d, *J =* 15.1 Hz,2H), 2.75 - 2.47 (m,8H), 2.37 - 2.23 (m,4H), 2.15 - 2.00 (m,5H), 1.87 - 1.76 (m, 1H), 1.63 - 1.56 (m,2H), 1.53 (s,5H), 1.46 (d, *J* = 11.0 Hz,30H), 1.38 - 1.25 (m,12H).

### (2) Synthesis of compound AAZTA-NI-FAPI-04

The compound 5 (29 mg, 0.02 mmol) was dissolved in 2 mL of trifluoroacetic acid, and stirred at room temperature for 2 hours. The reaction was monitored by mass spectrometry, the reaction mixture was subjected to rotary evaporation under reduced pressure to remove the solvent. The residue was dissolved in 2.9 mL of dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-25 min, 5%-100% B; 25-26 min, 100%-5% B; 26-30 min, 5% B, flow rate: 4 mL/min; ultraviolet: 280 nm). The target fraction was lyophilized by a lyophilizer to obtain the white solid AAZTA-NI-FAPI-04.

### Structural confirmation of compound AAZTA-NI-FAPI-04:

HRMS C₅₃H₇₀N₁₄O₁₆F₂ [M+H]⁺ calcd for 1197.5135, found 1197.5132.

### Step 2: Ga-68 labeling of AAZTA-NI-FAPI-04

A Ga-68 labeling route was as follows:

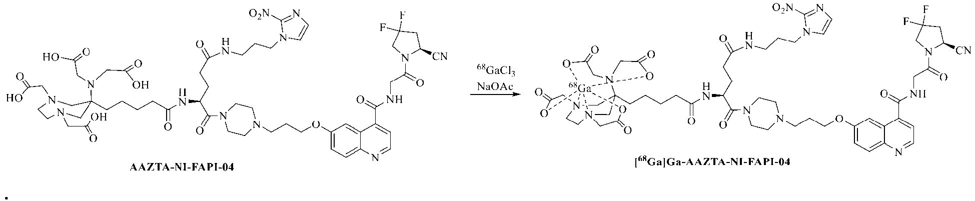

### A labeling method was as follows.

The AAZTA-NI-FAPI-04 obtained in the step 1 was prepared into a precursor solution with a concentration of 1 µg/µL from dimethyl sulfoxide, and 6 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 50°C for 10 minutes, and a radiochemical purity was determined by high-performance liquid chromatography equipped with a radioactivity detector, so as to obtain [⁶⁸Ga]Ga-AAZTA-NI-FAPI-04 with a radiochemical purity greater than 95%.

FIG. 3 shows a radioactive HPLC chromatogram of a labeling reaction solution of [⁶⁸Ga]Ga-AAZTA-NI-FAPI-04 prepared in Example 3 of the present invention, and the chromatogram indicates that the radiochemical purity of [⁶⁸Ga]Ga-AAZTA-NI-FAPI-04 is greater than 95%.

### Example 4: preparation of [¹⁷⁷Lu]Lu-AAZTA-NI-FAPI-04

### Step 1: synthesis of AAZTA-NI-FAPI-04

A synthetic route and a synthetic method were identical to those in the step 1 of Example 3.

### Step 2: Lu-177 labeling of AAZTA-NI-FAPI-04

A Lu-177 labeling route was as follows:

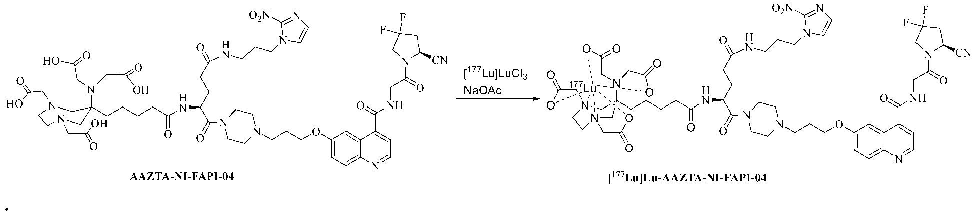

A labeling method was as follows.

The AAZTA-NI-FAPI-04 obtained in the step 1 was prepared into a precursor solution with a concentration of 1 µg/µL from dimethyl sulfoxide, and 24 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 50°C for 20 minutes, and a radiochemical purity was determined by high-performance liquid chromatography equipped with a radioactivity detector, so as to obtain [¹⁷⁷Lu]Lu-AAZTA-NI-FAPI-04 with a radiochemical purity greater than 95%.

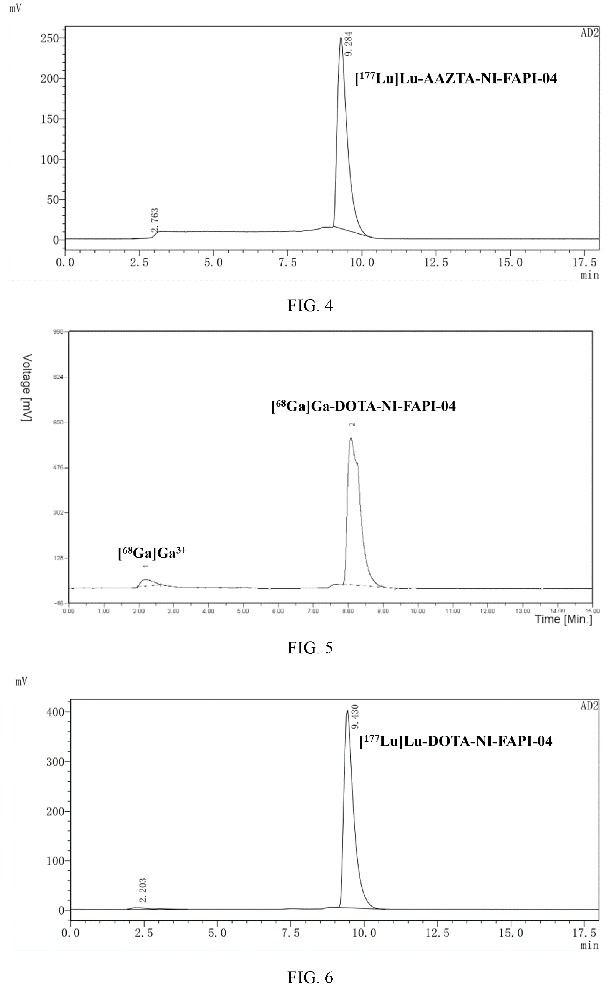

FIG. 4 shows a radioactive HPLC chromatogram of a labeling reaction solution of [¹⁷⁷Lu]Lu-AAZTA-NI-FAPI-04 prepared in Example 4 of the present invention, and the chromatogram indicates that the radiochemical purity of [¹⁷⁷Lu]Lu-AAZTA-NI-FAPI-04 is greater than 95%.

### Example 5: preparation of [⁶⁸Ga]Ga-DOTA-NI-FAPI-04

### Step 1: synthesis of DOTA-NI-FAPI-04

A synthetic route was as follows:

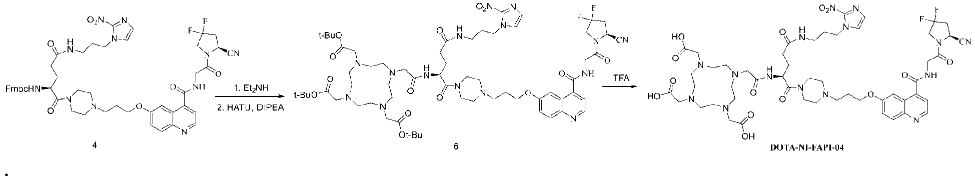

The synthetic route specifically included the following steps.

### (1) Synthesis of compound 6

The compound **4** (50.5 mg, 0.05 mmol) was dissolved in 2 mL of dichloromethane, added with 1 mL of diethylamine, and stirred at room temperature for 2 hours. The reaction was monitored by TLC, and the reaction mixture was subjected to rotary evaporation under reduced pressure to remove the solvent, so as to obtain a pale yellow oily intermediate. DOTA (*t*Bu)₃ (41 mg, 0.07 mmol) was dissolved in 2 mL of ultra-dry *N,N-*dimethylformamide, added with 2-(7-azabenzotriazol-1-yl)-*N*,*N*,*N'*,*N'*-tetramethyluronium hexafluorophosphate (27 mg, 0.07 mmol) and *N,N'*-diisopropylethylamine (13 mg, 0.1 mmol) under ice bath, and stirred under ice bath for 30 minutes to obtain a reaction mixture. The pale yellow oily intermediate (38 mg, 0.05 mmol) dissolved in 2 mL of ultra-dry *N,N-*dimethylformamide was added into the above reaction mixture to react at room temperature overnight, and washed with ethyl acetate (10 mL × 3) and saturated brine (10 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 10/1/0.1) to obtain the yellow oily compound **6** (21.5 mg, 0.02 mmol), with a yield of 40%.

### Structural confirmation of compound 6:

HRMS C₆₃H₉₃N₁₃O₁₄F₂Na [M+Na]⁺ calcd for 1344.6886, found 1344.6878;
¹H NMR (600 MHz, CD₃OD) δ 8.71 (d, *J* = 4.4 Hz,1H), 7.93 (d, *J* = 9.2 Hz,2H), 7.53 (t,2H), 7.41 (dd, *J =* 9.1, 2.6 Hz,1H), 7.10 (s,1H), 5.09 (dd, *J =* 9.3, 2.6 Hz,1H), 4.45 (t, *J* = 7.2 Hz,2H), 4.30 - 4.18 (m,5H), 4.15 - 4.06 (m,1H), 3.76 (d, *J* = 11.7 Hz,1H), 3.56 - 3.48 (m,1H), 3.40 (d, *J =* 5.6 Hz,2H), 3.27 (dt, *J =* 3.2, 1.6 Hz,5H), 3.20 - 3.14 (m,2H), 2.96 - 2.84 (m,3H), 2.76 (dd, *J =* 17.9, 8.9 Hz,3H), 2.69 - 2.52 (m,7H), 2.45 (d, *J =* 27.2 Hz,3H), 2.23 (t, *J =* 19.9, 12.6 Hz,3H), 2.12 - 1.96 (m,8H), 1.94 (d, *J =* 10.5 Hz,1H), 1.83 (dd, *J =* 13.9, 7.6 Hz,1H), 1.57 - 1.48 (m,2H), 1.47 - 1.38 (m,27H), 1.29 - 1.22 (m,8H).

### (2) Synthesis of compound DOTA-NI-FAPI-04

The compound **6** (21.5 mg, 0.02 mmol) was dissolved in 2 mL of trifluoroacetic acid, and stirred at room temperature for 2 hours. The reaction mixture was subjected to rotary evaporation under reduced pressure to remove the solvent. The residue was dissolved in 2.1 mL of dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-25 min, 5%-100% B; 25-26 min, 100%-5% B; 26-30 min, 5% B, flow rate: 4 mL/min; ultraviolet: 280 nm). The target fraction was lyophilized by a lyophilizer to obtain the white solid compound DOTA-NI-FAPI-04.

HRMS C₃₁H₆₉N₁₅O₁₄F₂ [M+H]⁺ calcd for 1154.5189, found 1154.5188.

### Step 2: Ga-68 labeling of DOTA-NI-FAPI-04

A ⁶⁸**Ga** labeling route was as follows:

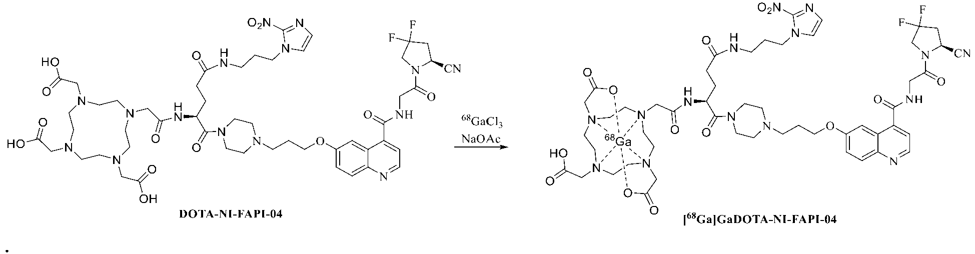

A labeling method was as follows.

The DOTA-NI-FAPI-04 obtained in the step 1 was prepared into a precursor solution with a concentration of 1 µg/µL from dimethyl sulfoxide, and 23 µL of the precursor solution was transferred into a 10 mL vial, and added with 72 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 95°C for 15 minutes, the reaction mixture was cooled to room temperature, and a radiochemical purity was determined by high-performance liquid chromatography equipped with a radioactivity detector, so as to obtain [⁶⁸Ga]Ga-DOTA-NI-FAPI-04 with a radiochemical purity greater than 95%.

FIG. 5 shows a radioactive HPLC chromatogram of a labeling reaction solution of [⁶⁸Ga]Ga-DOTA-NI-FAPI-04 prepared in Example 5 of the present invention, and the chromatogram indicates that the radiochemical purity of [⁶⁸Ga]Ga-DOTA-NI-FAPI-04 is greater than 95%.

### Example 6: preparation of [¹⁷⁷Lu]Lu- DOTA-NI-FAPI-04

### Step 1: synthesis of DOTA-NI-FAPI-04

A synthetic route and a synthetic method were identical to those in the step 1 of Example 5.

### Step 2: Lu-177 labeling of DOTA-NI-FAPI-04

A Lu-177 labeling route was as follows:

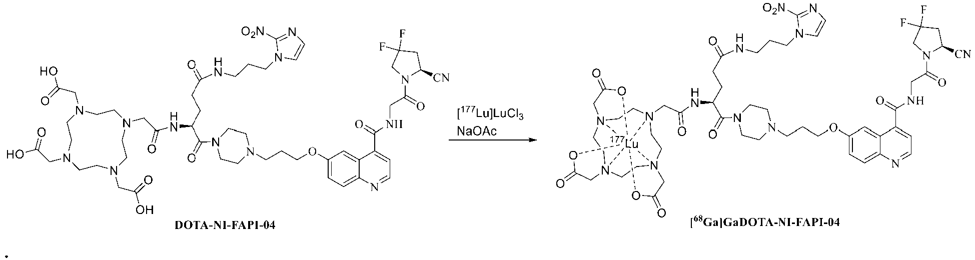

A labeling method was as follows.

The DOTA-NI-FAPI-04 obtained in the step 1 was prepared into a precursor solution with a concentration of 1 µg/µL from dimethyl sulfoxide, and 24 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 95°C for 20 minutes, and a radiochemical purity was determined by high-performance liquid chromatography equipped with a radioactivity detector, so as to obtain [¹⁷⁷Lu]Lu-DOTA-NI-FAPI-04 with a radiochemical purity greater than 95%.

FIG. 6 shows a radioactive HPLC chromatogram of a labeling reaction solution of [¹⁷⁷Lu]Lu-DOTA-NI-FAPI-04 prepared in Example 6 of the present invention, and the chromatogram indicates that the radiochemical purity of [¹⁷⁷Lu]Lu-DOTA-NI-FAPI-04 is greater than 95%.

### Example 7: preparation of [⁶⁸Ga]Ga-HBED-CC-NI-FAPI-04

### Step 1: synthesis of HBED-CC-NI-FAPI-04

A synthetic route was as follows:

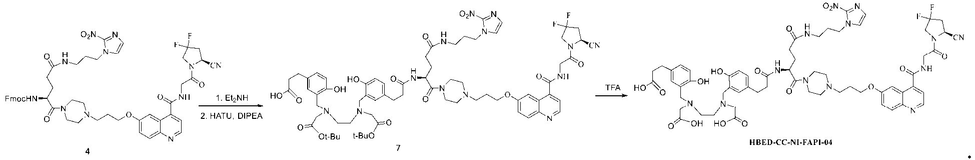

The synthetic route specifically included the following steps.

### (1) Synthesis of compound 7

The compound **4** (50.5 mg, 0.05 mmol) was dissolved in 2 mL of dichloromethane, added with 1 mL of diethylamine, and stirred at room temperature for 2 hours. The reaction was monitored by TLC, and the reaction mixture was subjected to rotary evaporation under reduced pressure to remove the solvent, so as to obtain a pale yellow oily intermediate. 3,3'-(((2,2,13,13-tetramethyl-4,11-dioxo-3,12-dioxa-6,9-diazatetradecane-6,9-diyl)bis(methylene ))bis(4-hydroxy-3,1-phenylene))dipropanoic acid (HBED-CC(*t*Bu)₂, 45 mg, 0.07 mmol) was dissolved in 2 mL of ultra-dry *N,N*-dimethylformamide, added with 2-(7-azabenzotriazol-1-yl)-*N*,*N*,*N'*,*N'*-tetramethyluronium hexafluorophosphate (27 mg, 0.07 mmol) and *N,N'*-diisopropylethylamine (13 mg, 0.1 mmol) under ice bath, and stirred under ice bath for 30 minutes to obtain a reaction mixture. The pale yellow intermediate (38 mg, 0.05 mmol) dissolved in 2 mL of ultra-dry *N*,*N-*dimethylformamide was added into the above reaction mixture to react at room temperature overnight, and washed with ethyl acetate (10 mL × 3) and saturated brine (10 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 8/1/0.1) to obtain the yellow oily compound **7** (21 mg, 0.02 mmol), with a yield of 40%.

### Structural confirmation of compound 7:

HRMS C₆₉H₉₀N₁₃O₁₆F₂ [M+H]⁺ calcd for 1394.6591, found 1394.6599;
¹H NMR (600 MHz, CD₃OD) δ 8.70 (t, *J* = 5.1 Hz,1H), 7.92 (dd, *J* = 11.9, 6.0 Hz1H), 7.52 (t, *J =* 4.2 Hz,1H), 7.45 - 7.39 (m,1H), 7.08 (d, *J =* 4.9 Hz,1H), 6.97 (dd, *J =* 12.5, 5.0 Hz,1H), 6.89 - 6.81 (m,1H), 6.66 (t, *J* = 9.0 Hz,1H), 5.08 (t, *J =* 11.8 Hz,1H), 4.45 (dd, *J* = 13.5, 6.7 Hz,2H), 4.30 - 4.23 (m,3H), 4.22 - 4.17 (m,1H), 4.14 - 4.05 (m,1H), 3.69 (d, *J =* 6.3 Hz,3H), 3.64 (d, *J =* 6.8 Hz,3H), 3.60 - 3.50 (m,2H), 3.27 (dt, *J =* 3.2, 1.6 Hz,7H), 3.26 - 3.23 (m,3H), 3.15 (dt, *J=* 13.9, 6.6 Hz,1H), 2.81 - 2.71 (m,6H), 2.68 (dd, *J* = 15.3, 8.3 Hz,2H), 2.55 (dd, *J* = 24.7, 17.2 Hz,4H), 2.45 (dt, *J =* 16.3, 7.4 Hz,3H), 2.22 - 2.12 (m,2H), 2.07 (dd, *J =* 13.3, 6.4 Hz,2H), 2.03 - 1.92 (m,3H), 1.77 - 1.64 (m,1H), 1.60 - 1.48 (m, 1H), 1.45 - 1.38 (m,14H), 1.33 - 1.22 (m,15H).

### (2) Synthesis of compound HBED-CC-NI-FAPI-04

The compound 7 (21 mg, 0.02 mmol) was dissolved in 2 mL of trifluoroacetic acid, and stirred at room temperature for 2 hours. The reaction mixture was subjected to rotary evaporation under reduced pressure to remove the solvent. The residue was dissolved in 2.1 mL of dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-25 min, 5%-100% B; 25-26 min, 100%-5% B; 26-30 min, 5% B, flow rate: 4 mL/min; ultraviolet: 280 nm). The target fraction was lyophilized by a lyophilizer to obtain the white solid compound HBED-CC-NI-FAPI-04.

### Structural confirmation of compound HBED-CC-NI-FAPI-04:

HRMS C₆₁H₇₃F₂N₁₃O₁₆ [M+H]⁺ calcd for 1282.5339, found 1282.5331.

### Step 2: Ga-68 labeling of HBED-CC-NI-FAPI-04

A Ga-68 labeling route was as follows:

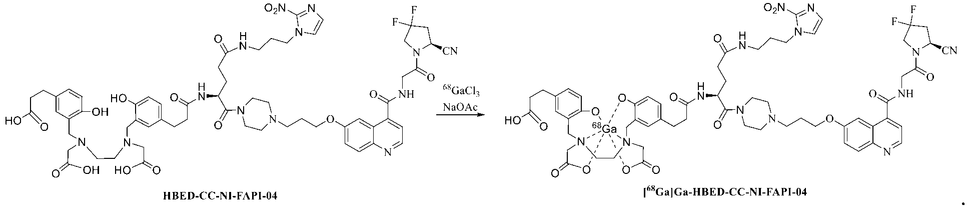

A labeling method was as follows.

The HBED-CC-NI-FAPI-04 obtained in the step 1 was prepared into a precursor solution with a concentration of 1 µg/µL from dimethyl sulfoxide, and 13 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 50°C for 10 minutes, the reaction mixture was cooled to room temperature, and a radiochemical purity was determined by high-performance liquid chromatography equipped with a radioactivity detector, so as to obtain [⁶⁸Ga]Ga-HBED-CC-NI-FAPI-04 with a radiochemical purity greater than 95%.

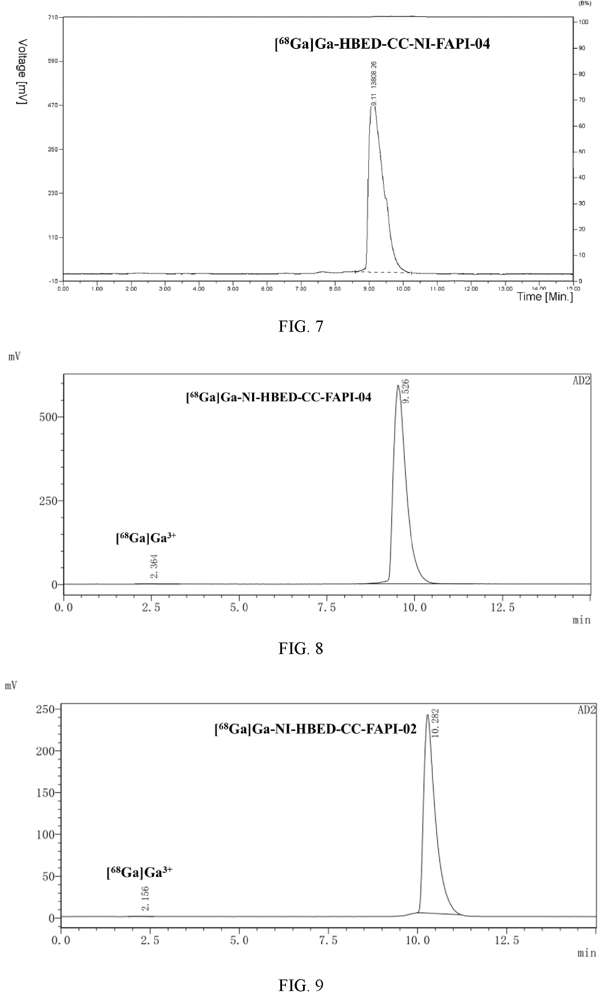

FIG. 7 shows a radioactive HPLC chromatogram of a labeling reaction solution of [⁶⁸Ga]Ga-HBED-CC-NI-FAPI-04 prepared in Example 7 of the present invention, and the chromatogram indicates that the radiochemical purity of [⁶⁸Ga]Ga-HBED-CC-NI-FAPI-04 is greater than 95%.

### Example 8: preparation of [⁶⁸Ga]Ga-NI-HBED-CC-FAPI-04

### Step 1: synthesis of NI-HBED-CC-FAPI-04

A synthetic route was as follows:

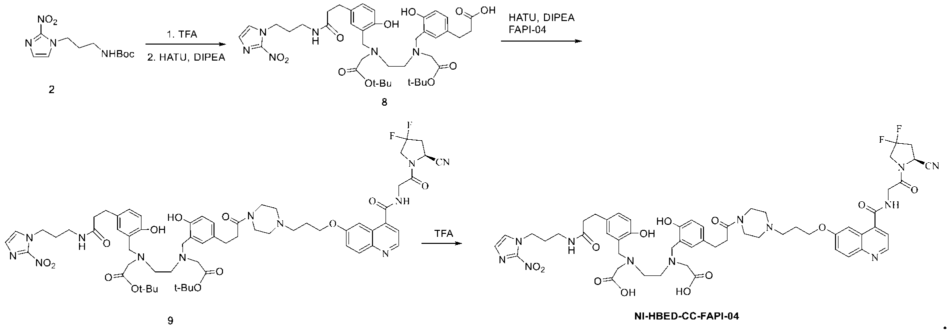

The synthetic route specifically included the following steps.

### (1) Synthesis of compound 8

The compound **2** (130 mg, 0.48 mmol) was dissolved in 2 mL of trifluoroacetic acid solution, stirred at room temperature for 30 minutes, and subjected to rotary evaporation under reduced pressure to remove the solvent, so as to obtain a white solid intermediate. 3,3'-(((2,2,13,13-tetramethyl-4,11-dioxo-3,12-dioxa-6,9-diazatetradecane-6,9-diyl)bis(methylene ))bis(4-hydroxy-3,1-phenylene))dipropanoic acid (HBED-CC(*t*Bu)₂, 309 mg, 0.48 mmol) was dissolved in 8 mL of ultra-dry *N,N*-dimethylformamide, added with 2-(7-azabenzotriazol-1-yl)-*N*,*N*,*N'*,*N'*-tetramethyluronium hexafluorophosphate (183 mg, 0.48 mmol) and N,N'-diisopropylethylamine (187 mg, 1.45 mmol) under ice bath, and stirred under ice bath for 30 minutes to obtain a reaction mixture. The white solid intermediate dissolved in 2 mL of ultra-dry *N,N*-dimethylformamide was added into the above reaction mixture to react at room temperature for 5 hours, and the resulting reaction mixture was washed with ethyl acetate (20 mL × 3) and saturated brine (20 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 10/1/0.1) to obtain the pale yellow oily compound **8** (55 mg, 0.07 mmol), with a yield of 15%.

### Structural confirmation of compound 8:

HRMS C₄₀H₅₆N₆O₁₁ [M+H]⁺ calcd for 797.4085, found 797.4081;
¹H NMR (400 MHz, CD₃OD ) δ 7.37 (s,1H), 7.08 (d, *J =* 1.0 Hz,1H), 7.01 (dd, *J =* 8.2, 1.9 Hz,2H), 6.92 (d, *J =* 2.6 Hz,2H), 6.69 (d, *J =* 2.8 Hz, ,1H), 6.67 (d, *J =* 2.8 Hz,1H), 4.27 (t, *J* = 7.1 Hz, 2H), 4.05 (dd,1H), 3.82 (s,2H), 3.75 (s,2H), 3.35 (s,2H), 3.31 (s,2H), 3.26 (dt, *J* = 3.3, 1.6 Hz,2H), 3.15 (t, *J* = 6.6 Hz,2H), 2.88 (dd, *J =* 7.8, 3.9 Hz,4H), 2.76 (dd, *J =* 16.5, 7.9 Hz,4H), 2.47 (t, *J* = 7.7 Hz,2H), 2.41 (t, *J* = 7.5 Hz,2H), 1.96 (d, *J* = 0.5 Hz,2H), 1.95 - 1.87 (m,2H), 1.42 (d, *J =* 1.8 Hz,18H).

### (2) Synthesis of compound 9

The compound **8** (54.7 mg, 0.069 mmol) was dissolved in 5 mL of ultra-dry *N,N*-dimethylformamide, added with 2-(7-azabenzotriazol-1-yl)-*N*,*N*,*N'*,*N'*-tetramethyluronium hexafluorophosphate (26 mg, 0.069 mmol) and *N,N'*-diisopropylethylamine (27 mg, 0.069 mmol) under ice bath, and stirred under ice bath for 30 minutes to obtain a reaction mixture. (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide (CAS: 2374782-82-6, 33.6 mg, 0.069 mmol) deprotected with trifluoroacetic acid and dissolved in 2 mL of ultra-dry *N,N*-dimethylformamide was added into the above reaction mixture. The reaction was carried out at room temperature for 5 hours, and the reaction mixture was washed with ethyl acetate (10 mL × 3) and saturated brine (10 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 10/1/0.1) to obtain the pale yellow oily compound **9** (31.7 mg, 0.025 mmol), with a yield of 36%.
HRMS C₆₄H₈₂N₁₂O₁₄₃F₂ [M+H]⁺ calcd for 1265.617, found 1265.6169;
¹H NMR (400 MHz, CD₃OD) δ 8.69 (d, *J =* 4.5 Hz, 1H), 7.94 - 7.87 (m, 2H), 7.51 (d, *J* = 4.4 Hz, 1H), 7.40 (d, *J* = 2.7 Hz, 1H), 7.39 - 7.3 5 (m, 1H), 7.10 - 7.04 (m, 1H), 7.02 - 6.94 (m, 2H), 6.88 (dd, *J*= 11.9, 1.7 Hz, 2H), 6.67 (dd, *J =* 12.9, 8.2 Hz, 2H), 5.09 (dd, *J =* 9.3, 3.0 Hz, 1H), 4.32 - 4.17 (m, 7H), 3.74 (s, 2H), 3.68 (s, 2H), 3.59 (s, 2H), 3.47 (s, 2H), 3.29 - 3.24 (m, 7H), 3.13 (t, *J =* 6.5 Hz, 2H), 2.82 - 2.68 (m, 11H), 2.63 - 2.54 (m, 4H), 2.48 (s, 2H), 2.39 (t, *J =* 7.5 Hz, 2H), 2.11 - 2.01 (m, 2H), 1.94 - 1.85 (m, 2H), 1.41 (s, 18H).

### (3) Synthesis of compound NI-HBED-CC-FAPI-04

The compound **9** (31.7 mg, 0.025 mmol) was dissolved in 2 mL of trifluoroacetic acid, and stirred at room temperature for 30 minutes. The reaction mixture was subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-18 min, 5%-59% B; 18-20 min, 59% B; 20-21 min, 59%-5% B; 21-25 min, 5% B, flow rate: 4 mL/min; ultraviolet: 280 nm). The target fraction was lyophilized by a lyophilizer to obtain the white solid NI-HBED-CC-FAPI-04.

### Structural confirmation of compound NI-HBED-CC-FAPI-04:

HRMS C₃₆H₆₇N₁₂O₁₃F₂ [M+H]⁺ calcd for 1153.4913, found 1153.4912.

### Step 2: Ga-68 labeling of NI-HBED-CC-FAPI-04

A Ga-68 labeling route was as follows:

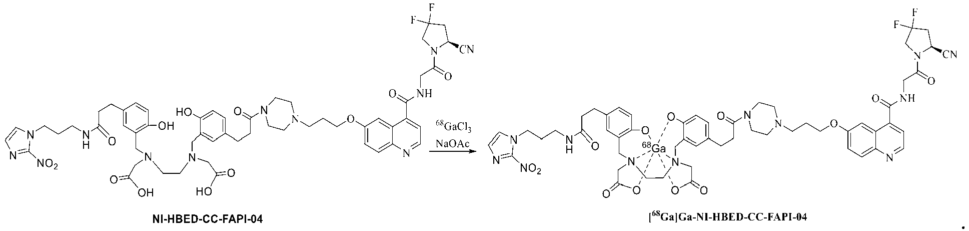

A labeling method was as follows.

The NI-HBED-CC-FAPI-04 obtained in the step 1 was prepared into a precursor solution with a concentration of 1 µg/µL from dimethyl sulfoxide, and 12 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 50°C for 10 minutes, the reaction mixture was cooled to room temperature, and a radiochemical purity was determined by high-performance liquid chromatography equipped with a radioactivity detector, so as to obtain [⁶⁸Ga]Ga-NI-HBED-CC-FAPI-04 with a radiochemical purity greater than 95%.

FIG. 8 shows a radioactive HPLC chromatogram of a labeling reaction solution of [⁶⁸Ga]Ga-NI-HBED-CC-FAPI-04 prepared in Example 8 of the present invention, and the chromatogram indicates that the radiochemical purity of [⁶⁸Ga]Ga-NI-HBED-CC-FAPI-04 is greater than 95%.

### Example 9: preparation of [⁶⁸Ga]Ga-NI-HBED-CC-FAPI-02

### Step 1: synthesis of NI-HBED-CC-FAPI-02

A synthetic route was as follows:

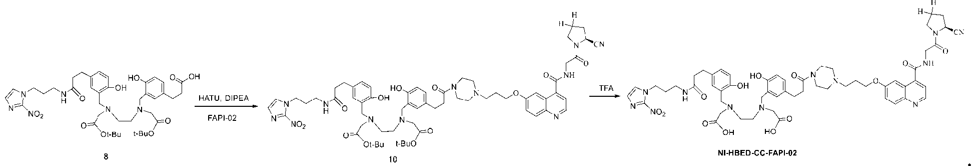

The synthetic route specifically included the following steps.

### (1) Synthesis of compound 10

(*S*)-6-[3-(4-Boc-1-piperazinyl)propoxy]-N-[2-(2-cyanopyrrolidinyl)-2-oxoethyl]quinolin e-4-carboxamide (CAS: 2370952-97-7, 35 mg, 0.064 mmol) was dissolved in 3 mL of trifluoroacetic acid, stirred at room temperature for 30 minutes, and subjected to rotary evaporation under reduced pressure to remove the solvent, so as to obtain an orange-red intermediate. The compound **8** was dissolved in 3 mL of ultra-dry *N,N*-dimethylformamide, added with 2-(7-azabenzotriazol-1-yl)-*N*,*N*,*N'*,*N'*-tetramethyluronium hexafluorophosphate (49 mg, 0.13 mmol) and *N,N*'-diisopropylethylamine (33 mg, 0.26 mmol) under ice bath, and stirred under ice bath for 30 minutes to obtain a reaction mixture. The orange-red intermediate dissolved in 2 mL of ultra-dry *N,N*-dimethylformamide was added into the above reaction mixture to react at room temperature for 5 hours, and the reaction mixture was washed with ethyl acetate (10 mL × 3) and saturated brine (10 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 8/1/0.1) to obtain the pale yellow oily compound **10** (38.4 mg, 0.031 mmol), with a yield of 48%.

### Structural confirmation of compound 10:

HRMS C₆₄H₈₅N₁₂O₁₃ [M+H]⁺ calcd for 1229.6353, found 1229.6356.

### (2) Synthesis of compound NI-HBED-CC-FAPI-02

The compound **10** (38.4 mg, 0.031 mmol) was dissolved in 2 mL of trifluoroacetic acid, and stirred at room temperature for 1 hour. The reaction mixture was subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-25 min, 5%-80% B; 25-26 min, 80%-5% B; 26-30 min, 5% B, flow rate: 4 mL/min; ultraviolet: 280 nm). The target fraction was lyophilized by a lyophilizer to obtain the white solid compound NI-HBED-CC-FAPI-02.

### Structural confirmation of compound NI-HBED-CC-FAPI-02:

HRMS C₃₆H₆₈N₁₂O₁₃ [M+H]⁺ calcd for 1117.5101, found 1117.5104.

### Step 2: Ga-68 labeling of NI-HBED-CC-FAPI-02

A Ga-68 labeling route was as follows:

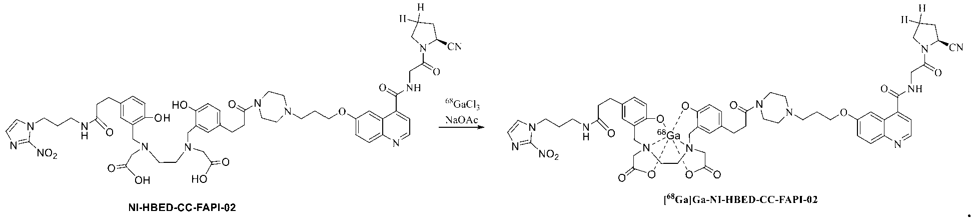

A labeling method was as follows.

The NI-HBED-CC-FAPI-02 obtained in the step 1 was prepared into a precursor solution with a concentration of 1 µg/µL from dimethyl sulfoxide, and 11 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at 50°C for 10 minutes, the reaction mixture was cooled to room temperature, and a radiochemical purity was determined by high-performance liquid chromatography equipped with a radioactivity detector, so as to obtain [⁶⁸Ga]Ga-NI-HBED-CC-FAPI-02 with a radiochemical purity greater than 95%.

FIG. 9 shows a radioactive HPLC chromatogram of a labeling reaction solution of [⁶⁸Ga]Ga-NI-HBED-CC-FAPI-02 prepared in Example 9 of the present invention, and the chromatogram indicates that the radiochemical purity of [⁶⁸Ga]Ga-NI-HBED-CC-FAPI-02 is greater than 95%.

### Example 10: preparation DOTA-[⁶⁸Ga]Ga-HBED-CC-FAPI-02

### Step 1: synthesis of DOTA-HBED-CC-FAPI-02

A synthetic route was as follows:

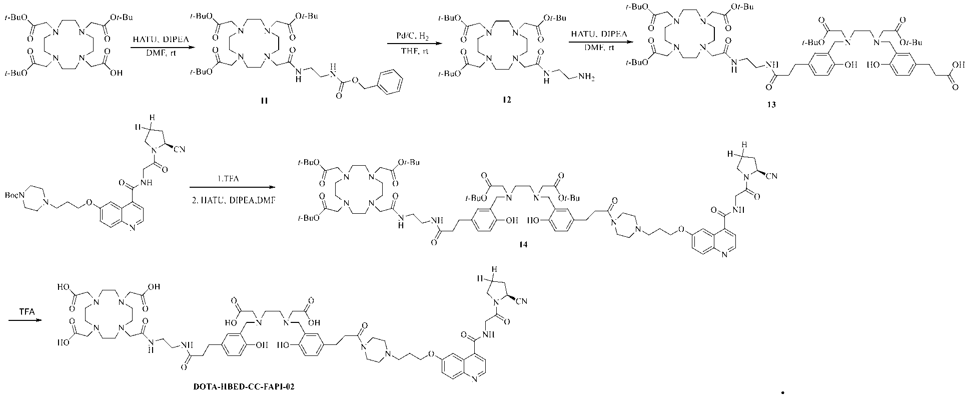

The synthetic route specifically included the following steps.

### (1) Synthesis of compound 11

In a 100 mL round-bottom flask, 2-(4,7,10-tris(2-(tert-butoxy)-2-oxoethyl)-1,4,7,10-tetraazacyclododecan-1-yl)acetic acid (DOTA(*t*Bu)₃, 1 g, 1.75 mmol) was dissolved in 15 mL of ultra-dry *N,N*-dimethylformamidee, added with 2-(7-azabenzotriazol-1-yl)-*N*,*N*,*N'*,*N'*-tetramethyluronium hexafluorophosphate (666 mg, 1.75 mmol) and *N,N'*-diisopropylethylamine (679 mg, 5.25 mmol) under ice bath, and stirred under ice bath for 30 minutes to obtain a reaction mixture. *N*-Cbz-1,2-ethanediamine (408 mg, 2.1 mmol) dissolved in 5 mL of ultra-dry *N*,*N*-dimethylformamide was added into the above reaction mixture to react at room temperature for 5 hours, and washed with ethyl acetate (20 mL × 3) and saturated brine (20 mL × 2). The organic phase was collected, dried with anhydrous sodium sulfate, and filtered to remove the anhydrous sodium sulfate, and the filtrate was subjected to rotary evaporation under reduced pressure to remove the solvent. The residue was purified by a flash chromatograph (dichloromethane/methanol/ammonia water, v/v/v = 20/1/0.1) to obtain the yellow solid compound **11** (1.02 g, 1.36 mmol), with a yield of 78%.

### Structural confirmation of compound 11:

HRMS C₃₈H₆₅N₆O₉ [M+H]⁺ calcd for 749.4807, found 749.4802;
¹H NMR (400 MHz, CDCl₃) δ 7.34 (d, *J =* 1.9 Hz, 4H), 7.32 (s, 1H), 6.58 (t, *J* = 4.7 Hz, 1H), 5.07 (s, 2H), 3.33 (s, 8H), 2.80 (s, 4H), 1.45 (s, 16H), 1.42 (s, 27H).

### (2) Synthesis of compound 12

In a 100 mL round-bottom flask, the compound **11** (1.02 g, 1.36 mmol) was dissolved in a mixed solution of 20 mL of tetrahydrofuran and 10 mL of methanol, and added with 10% Pd/C (150 mg, 0.14 mmol). The mixture was stirred under hydrogen atmosphere at room temperature overnight. The resulting reaction mixture was filtered through celite, and the filtrate was subjected to rotary evaporation under reduced pressure to remove the solvent, so as to obtain the white solid compound **12** (852 mg, 1.38 mmol), with a yield of >99%. The product was used directly in the next step without further purification.

### (3) Synthesis of compound 13

In a 100 mL round-bottom flask, 3,3'-(((2,2,13,13-tetramethyl-4,11-dioxo-3,12-di-oxa-6,9-diazatetradecane-6,9-diyl)bis(methylene))bis(4-hydroxy-3,1-phenylene))dipropanoic acid (HBED-CC(*t*Bu)₂, 500 mg, 0.78 mmol) was dissolved in 20 mL of ultra-dry *N,N*-dimethylformamide, added with *N,N*-diisopropylethylamine (300 mg, 2.3 mmol) under ice bath, dropwise added with 2-(7-azabenzotriazol-1-yl)-*N*,*N*,*N'*,*N'*-tetramethyluronium hexafluorophosphate (289 mg, 0.76 mmol) dissolved in 10 mL of ultra-dry *N,N*-dimethylformamide, and stirred under ice bath for 30 minutes to obtain a reaction mixture. The compound **12** (479 mg, 0.78 mmol) dissolved in 10 mL of ultra-dry *N,N*-dimethylformamide was added into the above reaction mixture to react at room temperature for 5 hours, and subjected to rotary evaporation under increased pressure to remove most of the *N,N*-dimethylformamide. The residue was washed with ethyl acetate (20 mL × 3) and saturated brine (20 mL × 2). The organic phase was collected, dried with anhydrous sodium sulfate, and subjected to rotary evaporation under reduced pressure to remove the solvent. The residue was purified by a flash chromatograph (dichloromethane/methanol/ammonia water, v/v/v = 8/1/0.1) to obtain the colorless solid compound **13** (234 mg, 0.19 mmol), with a yield of 24%.

### Structural confirmation of compound 13:

HRMS C₆₄H₁₀₃N₈O₁₆ [M+H]⁺ calcd for 1241.7643, found 1241.7652;
¹H NMR (400 MHz, CDCl₃) δ 9.22 (s,1H), 6.97 (d, *J* = 7.9 Hz, 2H), 6.84 (s, 1H), 6.79 (s, 1H), 6.70 (dd, *J =* 12.0, 8.3 Hz, 2H), 6.60 (s, 1H), 6.45 (s, 1H), 3.65 (s, 4H), 3.23 (s, 10H), 3.12 - 2.63 (m, 16H), 2.63 - 2.52 (m, 4H), 2.51 - 2.38 (m, 4H), 2.09 (d, *J =* 44.2 Hz, 7H), 1.49 - 1.33 (m, 45H).

### (4) Synthesis of compound 14

In a 50 mL round-bottom flask, (*S*)-6-[3-(4-Boc-1-piperazinyl)propoxy]-*N*-[2-(2-cyano-pyrrolidinyl)-2-oxoethyl]quinoline-4-carboxamide CAS: 2370952-97-7, 21 mg, 0.038 mmol) was dissolved in 3 mL of trifluoroacetic acid, stirred at room temperature for 30 minutes, and subjected to rotary evaporation to remove the solvent, so as to obtain an orange-red intermediate. In a 50 mL round-bottom flask, the compound **13** (36 mg, 0.029 mmol) was dissolved in 5 mL of ultra-dry *N,N*-dimethylformamide, added with *N,N'*-diisopropylethylamine (15 mg, 0.12 mmol) and 2-(7-azabenzotriazol-1-yl)-*N*,*N*,*N'*,*N'*-tetramethyluronium hexafluorophosphate (14 mg, 0.037 mmol) under ice bath, and stirred under ice bath for 30 minutes to obtain a reaction mixture. The above orange-red intermediate was dissolved in 5 mL of ultra-dry *N,N*-dimethylformamide and added into the above reaction mixture to react at room temperature for 5 hours, and the reaction mixture was subjected to rotary evaporation under increased pressure to remove most of the *N,N*-dimethylformamide. The residue was washed with ethyl acetate (10 mL × 3) and saturated brine (10 mL × 2). The organic phase was collected, dried with anhydrous sodium sulfate, and subjected to rotary evaporation under reduced pressure to remove the solvent. The residue was purified by a flash chromatograph (dichloromethane/methanol/ammonia water, v/v/v = 8/1/0.1) to obtain the pale yellow oily compound **14** (15 mg, 0.009 mmol), with a yield of 31%.

### Structural confirmation of compound 14:

HRMS C₈₈H₁₃₃N₁₄O₁₈ [M+H]⁺ calcd for 1673.9916, found 1673.9922.

### (5) Synthesis of compound DOTA-HBED-CC-FAPI-02

In a 25 mL round-bottom flask, the compound **14** (15 mg, 0.009 mmol) was dissolved in 4 mL of trifluoroacetic acid, stirred at room temperature for 2.5 hours, and subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-15 min, 5%-59% B; 15-16 min, 59%-5% B; 16-20 min, 5% B, flow rate: 4 mL/min; ultraviolet: 280 nm). The target fraction was lyophilized by a lyophilizer to obtain the white solid compound DOTA-HBED-CC-FAPI-02, with a purity of >95% as determined by LC-MS.

### Structural confirmation of compound DOTA-HBED-CC-FAPI-02:

HRMS C₆₈H₉₃N₁₄O₁₈ [M+H]⁺ calcd for 1393.6786, found 1393.6784.

### Step 2: Ga-68 labeling of DOTA-HBED-CC-FAPI-02

A Ga-68 labeling route was as follows:

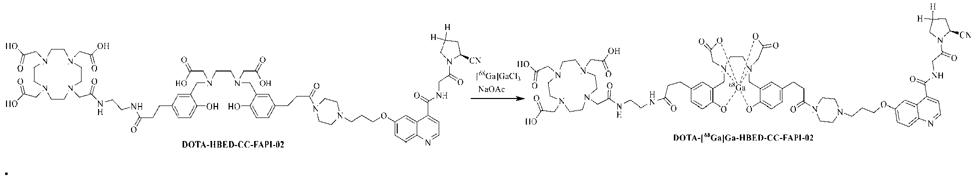

A labeling method was as follows.

The DOTA-HBED-CC-FAPI-02 obtained in the step 1 was prepared into a precursor solution with a concentration of 1 µg/µL from dimethyl sulfoxide, and 14 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at room temperature for 10 minutes to obtain DOTA-[⁶⁸Ga]Ga-HBED-CC-FAPI-02 with a radiochemical purity greater than 95%.

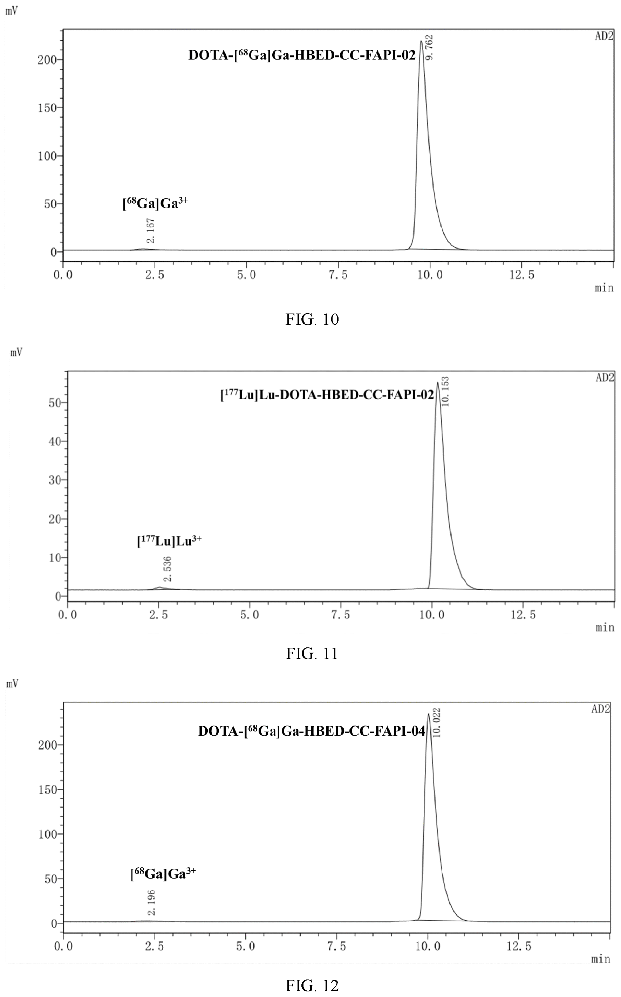

FIG. 10 shows a radioactive HPLC chromatogram of a labeling reaction solution of DOTA-[⁶⁸Ga]Ga-HBED-CC-FAPI-02 prepared in Example 10 of the present invention, and the chromatogram indicates that the radiochemical purity of DOTA-[⁶⁸Ga]Ga-HBED-CC-FAPI-02 is greater than 95%.

### Example 11: preparation of [¹⁷⁷Lu]Lu-DOTA-HBED-CC-FAPI-02

### Step 1: synthesis of DOTA-HBED-CC-FAPI-02

A synthetic route and a synthetic method were identical to those in the step 1 of Example 10.

### Step 2: Lu-177 labeling of DOTA-HBED-CC-FAPI-02

A Lu-177 labeling route was as follows:

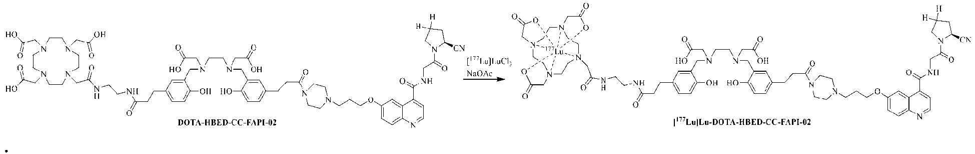

A labeling method was as follows.

The DOTA-HBED-CC-FAPI-02 obtained in the step 1 was prepared into a precursor solution with a concentration of 1 µg/µL from dimethyl sulfoxide, and 28 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 95°C for 20 minutes, and a radiochemical purity was determined by high-performance liquid chromatography equipped with a radioactivity detector, so as to obtain [¹⁷⁷Lu]Lu-DOTA-HBED-CC-FAPI-02 with a radiochemical purity greater than 95%.

FIG. 11 shows a radioactive HPLC chromatogram of a labeling reaction solution of [¹⁷⁷Lu]Lu-DOTA-HBED-CC-FAPI-02 prepared in Example 11 of the present invention, and the chromatogram indicates that the radiochemical purity of [¹⁷⁷Lu]Lu-DOTA-HBED-CC-FAPI-02 is greater than 95%.

### Example 12: preparation of DOTA-[⁶⁸Ga]Ga-HBED-CC-FAPI-04

### Step 1: synthesis of DOTA -HBED-CC-FAPI-04

A synthetic route was as follows:

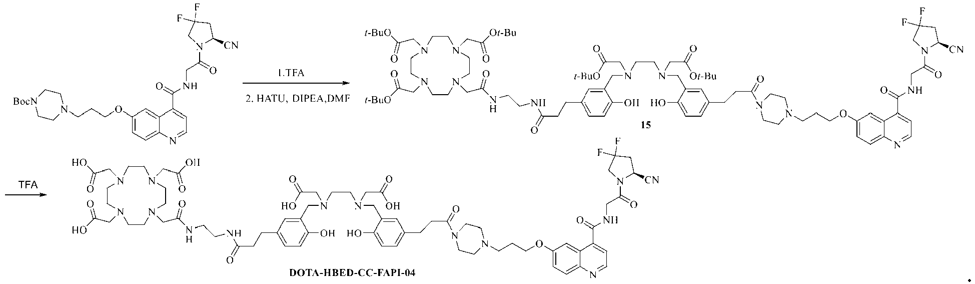

### (1) Synthesis of compound 15

In a 50 mL round-bottom flask, (*S*)-6-[3-(4-Boc-1-piperazinyl)pro-poxy]-*N*-[2-(2-cyano-4,4-difluoro-1-pyrrolidinyl)-2-oxoethyl]quinoline-4-carboxamide (CAS: 2374782-82-6, 25 mg, 0.043 mmol) was dissolved in 3 mL of trifluoroacetic acid, stirred at room temperature for 30 minutes, and subjected to rotary evaporation to remove the solvent, so as to obtain a yellow intermediate. In a 50 mL round-bottom flask, the compound **13** (31 mg, 0.025 mmol) was dissolved in 5 mL of ultra-dry N,N-dimethylformamide, added with *N,N*'-diisopropylethylamine (15 mg, 0.12 mmol) and 2-(7-azabenzotriazol-1-yl)-*N,N,N',N'*-tetramethyluronium hexafluorophosphate (15 mg, 0.039 mmol) under ice bath, and stirred under ice bath for 30 minutes to obtain a reaction mixture. The above yellow intermediate was dissolved in 5 mL of ultra-dry *N,N*-dimethylformamide and added into the above reaction mixture to react at room temperature for 5 hours, and the reaction mixture was subjected to rotary evaporation under increased pressure to remove most of the *N,N-*dimethylformamide. The residue was washed with ethyl acetate (10 mL × 3) and saturated brine (10 mL × 2). The organic phase was collected, dried with anhydrous sodium sulfate, and subjected to rotary evaporation under reduced pressure to remove the solvent. The residue was purified by a flash chromatograph (dichloromethane/methanol/ammonia water, v/v/v = 8/1/0.1) to obtain the pale yellow oily compound **15** (29 mg, 0.017 mmol), with a yield of 68%.

### Structural confirmation of compound 15:

HRMS C₈₈H₁₃₂N₁₄O₁₈F₂ [M+2H]²⁺ calcd for 855.49, found 855.4894.

### (2) Synthesis of compound DOTA-HBED-CC-FAPI-04

In a 25 mL round-bottom flask, the compound **15** (29 mg, 0.017 mmol) was dissolved in 4 mL of trifluoroacetic acid, stirred at room temperature for 2.5 hours, and subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-15 min, 5%-50% B; 15-16 min, 50%-5% B; 16-20 min, 5% B, flow rate: 4 mL/min; ultraviolet: 280 nm). The target fraction was lyophilized by a lyophilizer to obtain the white solid compound DOTA-HBED-CC-FAPI-04, with a purity of >95% as determined by LC-MS.

### Structural confirmation of compound DOTA-HBED-CC-FAPI-04:

HRMS C₆₈H₉₁N₁₄O₁₈F₂ [M+H]⁺ calcd for 1429.6598, found 1429.6592.

### Step 2: Ga-68 labeling of DOTA-HBED-CC-FAPI-04

A Ga-68 labeling route was as follows:

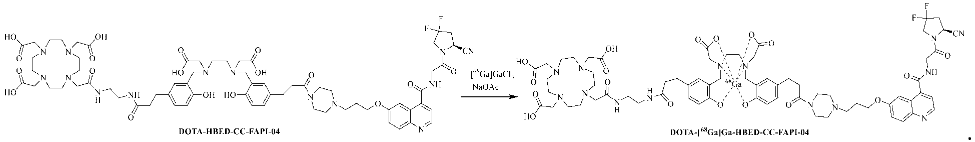

A labeling method was as follows.

The DOTA-HBED-CC-FAPI-04 obtained in the step 1 was prepared into a precursor solution with a concentration of 1 µg/µL from dimethyl sulfoxide, and 14 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at room temperature for 10 minutes to obtain DOTA-[⁶⁸Ga]Ga-HBED-CC-FAPI-04 with a radiochemical purity greater than 95%.

FIG. 12 shows a radioactive HPLC chromatogram of a labeling reaction solution of DOTA-[⁶⁸Ga]Ga-HBED-CC-FAPI-04 prepared in Example 12 of the present invention, and the chromatogram indicates that the radiochemical purity of DOTA-[⁶⁸Ga]Ga-HBED-CC-FAPI-04 is greater than 95%.

### Example 13: preparation of [¹⁷⁷Lu]Lu-DOTA-HBED-CC-FAPI-04

### Step 1: synthesis of DOTA-HBED-CC-FAPI-04

A synthetic route and a synthetic method were identical to those in the step 1 of Example 12.

### Step 2: Lu-177 labeling of DOTA-HBED-CC-FAPI-04

A Lu-177 labeling route was as follows:

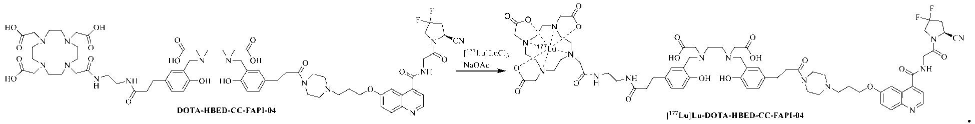

A labeling method was as follows.

The DOTA-HBED-CC-FAPI-04 obtained in the step 1 was prepared into a precursor solution with a concentration of 1 µg/µL from dimethyl sulfoxide, and 29 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 95°C for 20 minutes, and a radiochemical purity was determined by high-performance liquid chromatography equipped with a radioactivity detector, so as to obtain [¹⁷⁷Lu]Lu-DOTA-HBED-CC-FAPI-04 with a radiochemical purity greater than 95%.

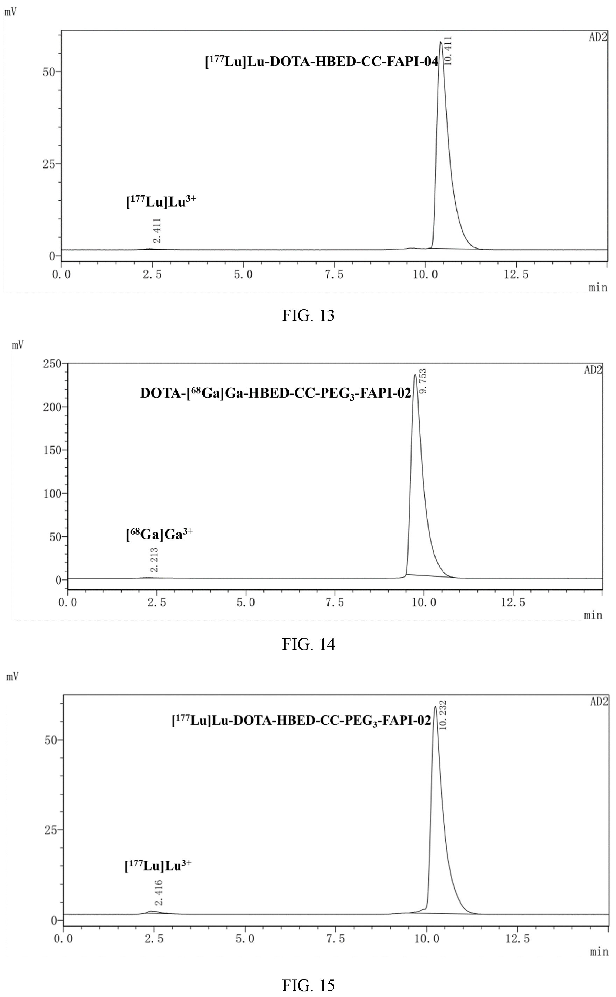

FIG. 13 shows a radioactive HPLC chromatogram of a labeling reaction solution of [¹⁷⁷Lu]Lu-DOTA-HBED-CC-FAPI-04 prepared in Example 13 of the present invention, and the chromatogram indicates that the radiochemical purity of [¹⁷⁷Lu]Lu-DOTA-HBED-CC-FAPI-04 is greater than 95%.

### Example 14: preparation of DOTA-[⁶⁸Ga]Ga-HBED-CC-PEG₃-FAPI-02

### Step 1: synthesis of DOTA -HBED-CC-PEG₃-FAPI-02

A synthetic route was as follows:

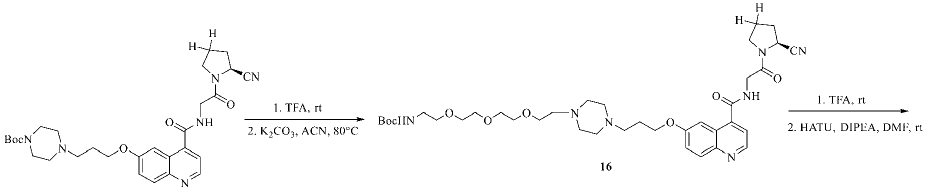

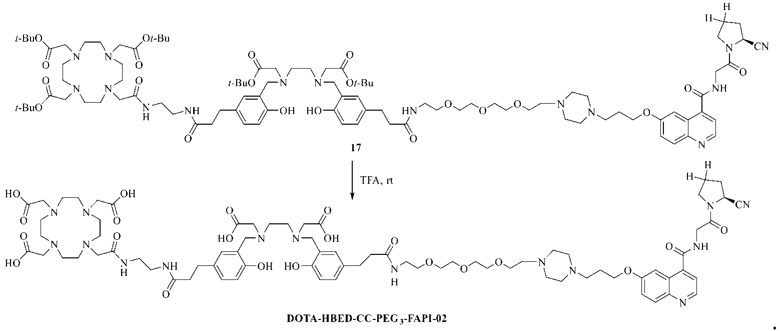

The synthetic route specifically included the following steps.

### (1) Synthesis of compound 16

In a 50 mL round-bottom flask, (*S*)-6-[3-(4-Boc-1-piperazinyl)propo-xy]-*N*-[2-(2-cyanopyrrolidinyl)-2-oxoethyl]quinoline-4-carboxamide (CAS: 2370952-97-7, 212 mg, 0.39 mmol) was dissolved in 3 mL of trifluoroacetic acid, stirred at room temperature for 30 minutes, subjected to rotary evaporation to remove the solvent, so as to obtain an orange-red substance. The orange-red substance was dissolved in 10 mL of anhydrous acetonitrile, added with anhydrous potassium carbonate (216 mg, 1.56 mmol), stirred at room temperature for 30 minutes, and then added with tert-butyl (2-(2-(2-(2-bromoethoxy)ethoxy)ethoxy)ethyl)carbamate (167 mg, 0.47 mmol). The mixture was refluxed at 80°C overnight and filtered through celite to remove the potassium carbonate, and the filtrate was subjected to rotary evaporation under reduced pressure to remove the solvent. The residue was purified by a flash chromatograph (dichloromethane/methanol/ammonia water, v/v/v = 10/1/0.1) to obtain the pale yellow oily compound **16**(191 mg, 0.26 mmol), with a yield of 67%.

### Structural confirmation of compound 16:

HRMS C₃₇H₅₆N₇O₈ [M+H]⁺ calcd for 726.419, found 726.4179;
¹H NMR (400 MHz, CDCl₃) δ 8.66 (d, *J =* 4.4 Hz, 1H), 7.92 (dd, *J =* 9.2, 4.3 Hz, 1H), 7.62 (d, *J =* 2.3 Hz, 1H), 7.51 (s, 1H), 7.41 (d, *J =* 4.4 Hz, 1H), 7.30 (dd, *J =* 9.2, 2.6 Hz, 1H), 4.74 (dd, *J =* 23.1, 6.2 Hz, 1H), 4.15 - 4.05 (m, 2H), 3.68 - 3.51 (m, 12H), 3.51 - 3.43 (m, 3H), 3.36 (s, 1H), 3.25 (d, *J =* 3.4 Hz, 2H), 2.62 (s, 12H), 2.21 (tdd, *J =* 17.5, 10.5, 7.9 Hz, 4H), 2.08 - 1.95 (m, 2H), 1.37 (s, 9H).

### (2) Synthesis of compound 17

In a 50 mL round-bottom flask, the compound **16** (32 mg, 0.044 mmol) was dissolved in 3 mL of trifluoroacetic acid, stirred at room temperature for 30 minutes, subjected to rotary evaporation to remove the solvent, so as to obtain an orange-yellow intermediate. In a 50 mL round-bottom flask, the compound **13** (37 mg, 0.03 mmol) was dissolved in 5 mL of ultra-dry *N,N-*dimethylformamide, added with *N,N*'-diisopropylethylamine (15 mg, 0.12 mmol) and 2-(7-azabenzotriazol-1-yl)-*N*,*N*,*N'*,*N'*-tetramethyluronium hexafluorophosphate (17 mg, 0.045 mmol) under ice bath, and stirred under ice bath for 30 minutes to obtain a reaction mixture. The above orange-yellow intermediate was dissolved in 5 mL of ultra-dry *N,N*-dimethylformamide and added into the above reaction mixture to react at room temperature for 5 hours, and the reaction mixture was subjected to rotary evaporation under increased pressure to remove most of the *N,N*-dimethylformamide. The residue was washed with ethyl acetate (10 mL × 3) and saturated brine (10 mL × 2). The organic phase was collected, dried with anhydrous sodium sulfate, and subjected to rotary evaporation under reduced pressure to remove the solvent. The residue was purified by a flash chromatograph (dichloromethane/methanol/ammonia water, v/v/v = 8/1/0.1) to obtain the pale yellow oily compound **17** (31 mg, 0.017 mmol), with a yield of 57%.

### Structural confirmation of compound 17:

HRMS C₉₆H₁₅₀N₁₅O₂₁Na [M+H+Na]²⁺ calcd for 936.0508, found 936.0511.

### (3) Synthesis of compound DOTA-HBED-CC-PEG₃-FAPI-02

In a 25 mL round-bottom flask, the compound **17** (31 mg, 0.017 mmol) was dissolved in 4 mL of trifluoroacetic acid, stirred at room temperature for 2.5 hours, and subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-15 min, 5%-50% B; 15-16 min, 50%-5% B; 16-20 min, 5% B, flow rate: 4 mL/min; ultraviolet: 280 nm). The target fraction was lyophilized by a lyophilizer to obtain the white solid compound DOTA-HBED-CC-PEG₃-FAPI-02, with a purity of >95% as determined by LC-MS.

### Structural confirmation of compound DOTA-HBED-CC-PEG₃-FAPI-02:

HRMS C₇₆H₁₁₀N₁₃O₂₁ [M+H]⁺ calcd for 1568.7995, found 1568.7989.

### Step 2: Ga-68 labeling of DOTA-HBED-CC-PEG₃-FAPI-02

A Ga-68 labeling route was as follows:

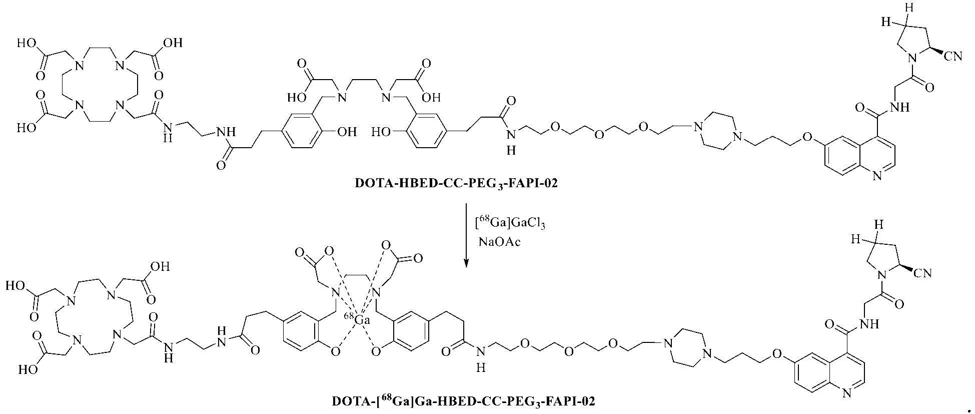

A labeling method was as follows.

The DOTA-HBED-CC-PEG₃-FAPI-02 obtained in the step 1 was prepared into a precursor solution with a concentration of 1 µg/µL from dimethyl sulfoxide, and 16 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at room temperature for 10 minutes to obtain DOTA-[⁶⁸Ga]Ga-HBED-CC-PEG₃-FAPI-02 with a radiochemical purity greater than 95%.

FIG. 14 shows a radioactive HPLC chromatogram of a labeling reaction solution of DOTA-[⁶⁸Ga]Ga-HBED-CC-PEG₃-FAPI-02 prepared in Example 14 of the present invention, and the chromatogram indicates that the radiochemical purity of DOTA-[⁶⁸Ga]Ga-HBED-CC-PEG₃-FAPI-02 is greater than 95%.

### Example 15: preparation of [¹⁷⁷Lu]Lu-DOTA-HBED-CC-PEG₃-FAPI-02

### Step 1: synthesis of DOTA-HBED-CC-PEG₃-FAPI-02

A synthetic route and a synthetic method were identical to those in the step 1 of Example 14.

### Step 2: Lu-177 labeling of DOTA-HBED-CC-PEG₃-FAPI-02

A Lu-177 labeling route was as follows:

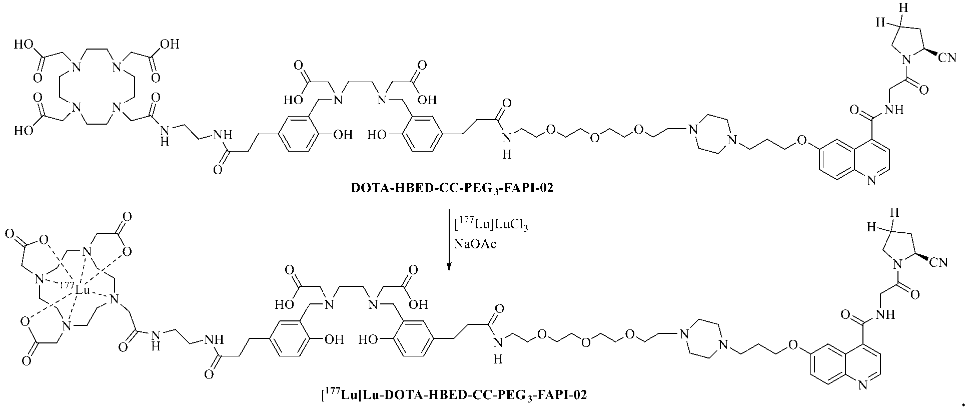

A labeling method was as follows.

The DOTA-HBED-CC-PEG₃-FAPI-02 obtained in the step 1 was prepared into a precursor solution with a concentration of 1 µg/µL from dimethyl sulfoxide, and 31 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 95°C for 20 minutes, and a radiochemical purity was determined by high-performance liquid chromatography equipped with a radioactivity detector, so as to obtain [¹⁷⁷Lu]Lu-DOTA-HBED-CC-PEG₃-FAPI-02 with a radiochemical purity greater than 95%.

FIG. 15 shows a radioactive HPLC chromatogram of a labeling reaction solution of [¹⁷⁷Lu]Lu-DOTA-HBED-CC-PEG₃-FAPI-02 prepared in Example 15 of the present invention, and the chromatogram indicates that the radiochemical purity of [¹⁷⁷Lu]Lu-DOTA-HBED-CC-PEG₃-FAPI-02 is greater than 95%.

### Example 16: preparation of DOTA-[⁶⁸Ga]Ga-HBED-CC-PEG₃-FAPI-04

### Step 1: synthesis of DOTA-HBED-CC-PEG₃-FAPI-04

A synthetic route was as follows:

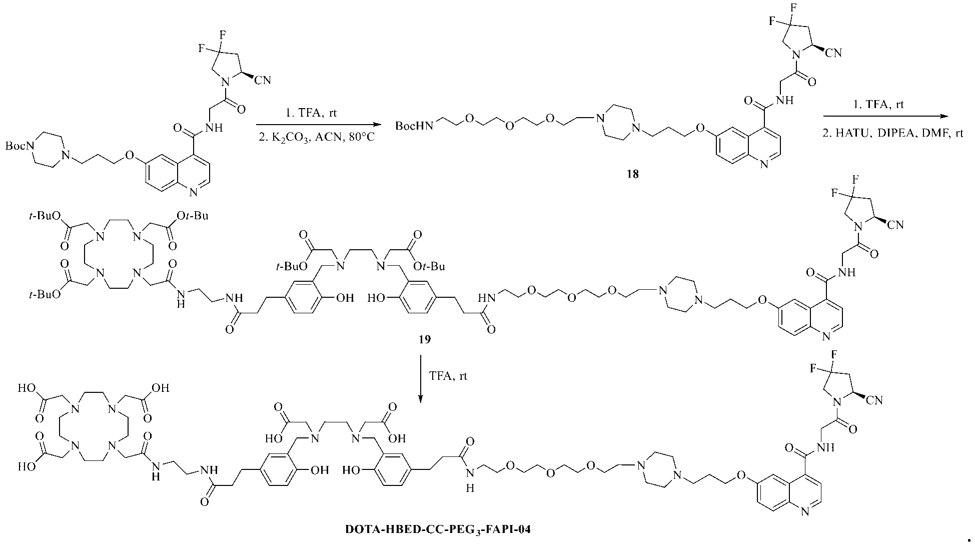

The synthetic route specifically included the following steps.

### (1) Synthesis of compound 18

In a 50 mL round-bottom flask, (S)-6-[3-(4-Boc-1-piperazinyl)propoxy]-N-[2-(2-cyano-4,4-difluoro-1-pyrrolidinyl)-2-oxoethyl]quinoline-4-carboxamide (CAS: 2374782-82-6, 105 mg, 0.18 mmol) was dissolved in 3 mL of trifluoroacetic acid, stirred at room temperature for 30 minutes, subjected to rotary evaporation to remove the solvent, so as to obtain a yellow substance. The yellow substance was dissolved in 10 mL of anhydrous acetonitrile, added with anhydrous potassium carbonate (106 mg, 0.77 mmol), stirred at room temperature for 30 minutes, and then added with tert-butyl (2-(2-(2-(2-bromoethoxy)ethoxy)ethoxy)ethyl)-carbamate (82 mg, 0.23 mmol). The mixture was refluxed at 80°C overnight and filtered through celite to remove the potassium carbonate, and the filtrate was subjected to rotary evaporation under reduced pressure to remove the solvent. The residue was purified by a flash chromatograph (dichloromethane/methanol/ammonia water, v/v/v = 10/1/0.1) to obtain the pale yellow oily compound **18** (131 mg, 0.17 mmol), with a yield of 94%.

### Structural confirmation of compound 18:

HRMS C₃₇H₃₄N₇O₈F₂ [M+H]⁺ calcd for 762.3996, found 762.3992;
¹H NMR (600 MHz, CDCl₃) δ 8.57 (d, *J =* 28.1 Hz, 1H), 7.85 (d, *J =* 5.6 Hz, 2H), 7.56 (d, *J* = 26.6 Hz, 1H), 7.33 (s, 1H), 5.26 (s, 1H), 4.93 (s, 1H), 4.22 (t, *J =* 21.4 Hz, 1H), 4.03 (d, *J* = 35.2 Hz, 4H), 3.95 - 3.78 (m, 2H), 3.65 - 3.33 (m, 13H), 3.20 (d, *J =* 21.6 Hz, 2H), 2.61 (d, *J* = 43.8 Hz, 13H), 1.99 (s, 2H), 1.44 - 1.26 (m, 9H).

### (2) Synthesis of compound 19

In a 50 mL round-bottom flask, the compound **18** (33 mg, 0.043 mmol) was dissolved in 3 mL of trifluoroacetic acid, stirred at room temperature for 30 minutes, and subjected to rotary evaporation to remove the solvent, so as to obtain a pale yellow intermediate. In a 50 mL round-bottom flask, the compound **13** (32 mg, 0.026 mmol) was dissolved in 5 mL of ultra-dry *N,N*-dimethylformamide, added with *N,N'*-diisopropylethylamine (13 mg, 0.10 mmol) and 2-(7-azabenzotriazol-1-yl)-*N*,*N*,*N'*,*N'*-tetramethyluronium hexafluorophosphate (13 mg, 0.034 mmol) under ice bath, and stirred under ice bath for 30 minutes to obtain a reaction mixture. The above pale yellow intermediate was dissolved in 5 mL of ultra-dry *N,N-*dimethylformamide and added into the above reaction mixture to react at room temperature for 5 hours, and the reaction mixture was subjected to rotary evaporation under increased pressure to remove most of the *N,N*-dimethylformamide. The residue was washed with ethyl acetate (10 mL × 3) and saturated brine (10 mL × 2). The organic phase was collected, dried with anhydrous sodium sulfate, and subjected to rotary evaporation under reduced pressure to remove the solvent. The residue was purified by a flash chromatograph (dichloromethane/methanol/ammonia water, v/v/v = 8/1/0.1) to obtain the pale yellow oily compound **19** (28 mg, 0.015 mmol), with a yield of 58%.

### Structural confirmation of compound 19:

HRMS C₉₆H₁₄₉N₁₃O₂₁F₂ [M+2H]²⁺ calcd for 943.0504, found 943.0520.

### (3) Synthesis of compound DOTA-HBED-CC-PEG₃-FAPI-04

In a 25 mL round-bottom flask, the compound **19** (28 mg, 0.015 mmol) was dissolved in 4 mL of trifluoroacetic acid, stirred at room temperature for 2.5 hours, and subjected to rotary evaporation under reduced pressure to remove the solvent. The residue was dissolved in dimethyl sulfoxide, and separated and purified by a semi-preparative chromatographic column (Eclipse XDB-C18, 5 µm, 9.4×250 mm) (phase A: 0.1% trifluoroacetic acid-water; phase B: 0.1% trifluoroacetic acid-acetonitrile; gradient: 0-15 min, 5%-50% B; 15-16 min, 50%-5% B; 16-20 min, 5% B, flow rate: 4 mL/min; ultraviolet: 280 nm). The target fraction was lyophilized by a lyophilizer to obtain the white solid compound DOTA-HBED-CC-PEG₃-FAPI-04, with a purity of >95% as determined by LC-MS.

### Structural confirmation of compound DOTA-HBED-CC-PEG₃-FAPI-04:

HRMS C₇₆H₁₀₈N₁₃O₂₁F₂ [M+H]⁺ calcd for 1604.7806, found 1604.7778.

### Step 2: Ga-68 labeling of DOTA-HBED-CC-PEG₃-FAPI-04

A Ga-68 labeling route was as follows:

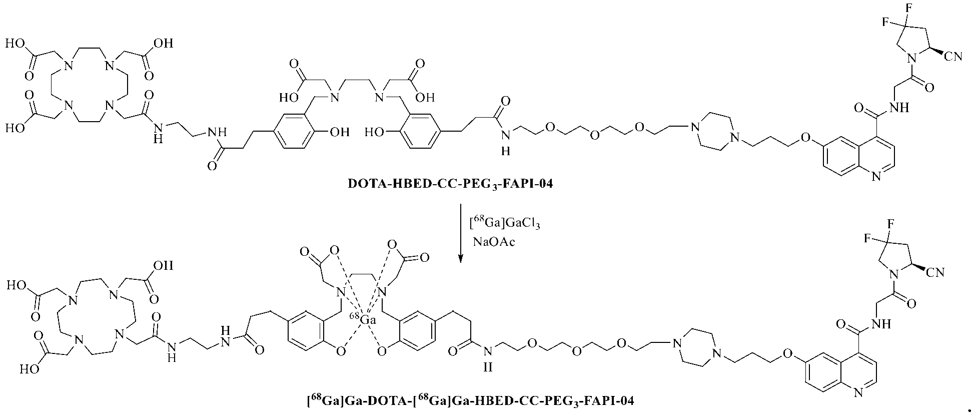

A labeling method was as follows.

The DOTA-HBED-CC-PEG₃-FAPI-04 obtained in the step 1 was prepared into a precursor solution with a concentration of 1 µg/µL from dimethyl sulfoxide, and 16 µL of the precursor solution was transferred into a 10 mL vial, and added with 135 µL of 3 M sodium acetate solution to obtain a radioligand sodium acetate mixture solution. A germanium-gallium generator (iThemba) was eluted with 6 mL of 0.6 M high-purity hydrochloric acid solution to obtain a [⁶⁸Ga]GaCl₃ hydrochloric acid solution, and 300 µL of the solution was added into the above radioligand sodium acetate mixture solution and mixed uniformly. Subsequently, the reaction was carried out at room temperature for 10 minutes to obtain DOTA-[⁶⁸Ga]Ga-HBED-CC-PEG₃-FAPI-04 with a radiochemical purity greater than 95%.

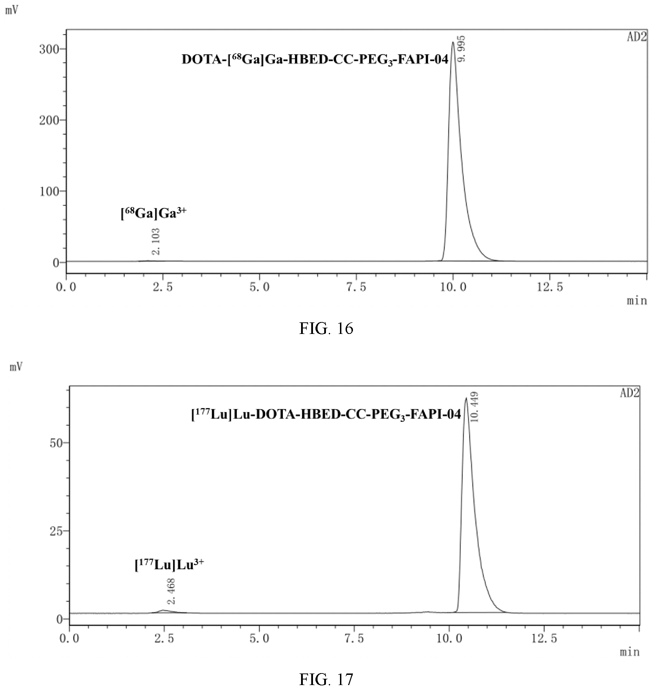

FIG. 16 shows a radioactive HPLC chromatogram of a labeling reaction solution of DOTA-[⁶⁸Ga]Ga-HBED-CC-PEG₃-FAPI-04 prepared in Example 16 of the present invention, and the chromatogram indicates that the radiochemical purity of DOTA-[⁶⁸Ga]Ga-HBED-CC-PEG₃-FAPI-04 is greater than 95%.

### Example 17: preparation of [¹⁷⁷Lu]Lu-DOTA-HBED-CC-PEG₃-FAPI-04

### Step 1: synthesis of DOTA-HBED-CC-PEG₃-FAPI-04

A synthetic route and a synthetic method were identical to those in the step 1 of Example 16.

### Step 2: Lu-177 labeling of DOTA-HBED-CC-PEG₃-FAPI-04

A Lu-177 labeling route was as follows:

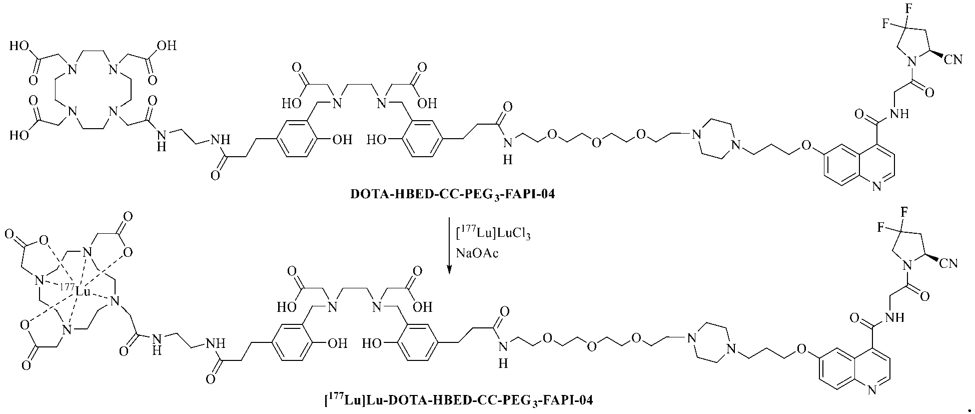

A labeling method was as follows.

The DOTA-HBED-CC-PEG₃-FAPI-04 obtained in the step 1 was prepared into a precursor solution with a concentration of 1 µg/µL from dimethyl sulfoxide, and 32 µL of the precursor solution was transferred into a 10 mL vial, added with 12 µL of 3 M sodium acetate solution, added with 400 µL of [¹⁷⁷Lu]LuCl₃ hydrochloric acid solution and mixed uniformly. Subsequently, the reaction was carried out at 95°C for 20 minutes, and a radiochemical purity was determined by high-performance liquid chromatography equipped with a radioactivity detector, so as to obtain [¹⁷⁷Lu]Lu-DOTA-HBED-CC-PEG₃-FAPI-04 with a radiochemical purity greater than 95%.

FIG. 17 shows a radioactive HPLC chromatogram of a labeling reaction solution of [¹⁷⁷Lu]Lu-DOTA-HBED-CC-PEG₃-FAPI-04 prepared in Example 17 of the present invention, and the chromatogram indicates that the radiochemical purity of [¹⁷⁷Lu]Lu-DOTA-HBED-CC-PEG₃-FAPI-04 is greater than 95%.

### Application Example 1

Cellar uptake of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in HT1080-FAP cells in vitro:
HT1080-FAP cells were prepared into a cell suspension at a density of 1.25×10⁶ cells/mL, and 400 µL of the suspension was respectively seeded into a 6-well plate and cultured for 24 hours. The well plates of groups were respectively added with solutions of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 at 37-111 KBq, and incubated in a constant-temperature incubator at 37°C. At 10, 30, 60, 90 and 120 minutes of incubation of each group, the cellar uptake was terminated with a PBS solution, the cells were lysed with 1 M NaOH, the cell lysate was absorbed onto filter paper, and the filter paper was placed into a plastic test tube for radioactivity counting. For a blocking experiment, an excess of unlabeled DOTA-FAPI-04 was respectively co-incubated with a corresponding radiolabeled drug. After 60 minutes of incubation, the cellar uptake was terminated with a PBS solution, the cells were lysed with 1 M NaOH, the cell lysate was absorbed onto filter paper, and the filter paper was placed into a plastic test tube for radioactivity counting.

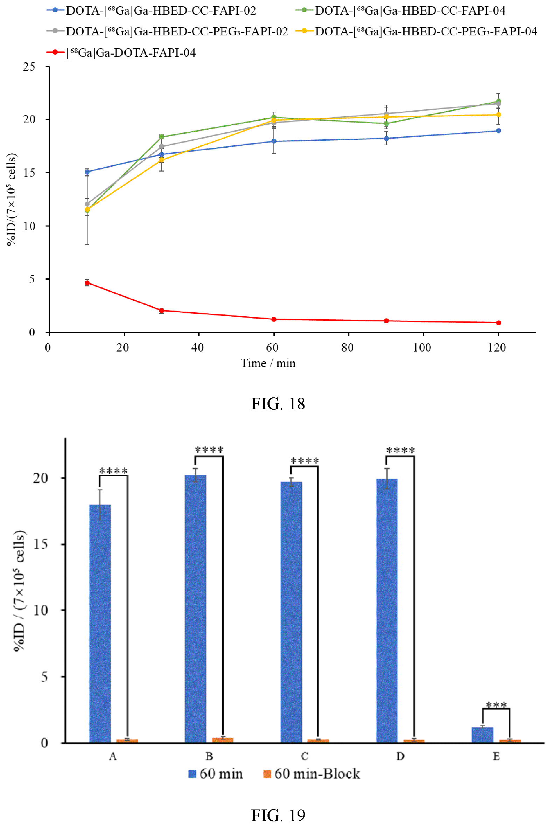

FIG. 18 shows uptake-time curves of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in HT1080-FAP cells in vitro in Application Example 1 of the present invention (n=3). FIG. 19 shows uptake and blocking results at 60 minutes of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in HT1080-FAP cells in vitro in Application Example 1 of the present invention (n=3).

Results of the in-vitro cellular uptake experiment indicated that the uptake of DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02 and DOTA-[⁶⁸Ga]Ga-HBED-CC-PEG₃-FAPI-04 in FAP-positive HT1080-FAP cells was gradually increased with prolonged incubation time, and uptake values of test drugs at each time point monitored were all higher than that of the positive control [⁶⁸Ga]Ga-DOTA-FAPI-04. The uptake of all drugs could be blocked by an excess of DOTA-FAPI-04, which indicated that all drugs specifically targeted the FAP.

### Application Example 2

Uptake of [¹⁷⁷Lu]Lu-DOTA-HBED-CC-FAPI-02, [¹⁷⁷Lu]Lu-DOTA-HBED-CC-FAPI-04, [¹⁷⁷Lu]Lu-DOTA-HBED-CC-PEG₃-FAPI-02, [¹⁷⁷Lu]Lu-DOTA-HBED-CC-PEG₃-FAPI-04 and [¹⁷⁷Lu]Lu-DOTA-FAPI-04 in HT1080-FAP cells in vitro:
HT1080-FAP cells were prepared into a cell suspension at a density of 1.25×10⁶ cells/mL, and 400 µL of the suspension was respectively seeded into a 6-well plate and cultured for 24 hours. The well plates of groups were respectively added with solutions of [¹⁷⁷Lu]Lu-DOTA-HBED-CC-FAPI-02, [¹⁷⁷Lu]Lu-DOTA-HBED-CC-FAPI-04, [¹⁷⁷Lu]Lu-DOTA-HBED-CC-PEG₃-FAPI-02, [¹⁷⁷Lu]Lu-DOTA-HBED-CC-PEG₃-FAPI-04 and [¹⁷⁷Lu]Lu-DOTA-FAPI-04 at 81 KBq, and incubated in a constant-temperature incubator at 37°C. At 10, 30, 60, 90 and 120 minutes of incubation of each group, the cellar uptake was terminated with a PBS solution, the cells were lysed with 1 M NaOH, the cell lysate was absorbed onto filter paper, and the filter paper was placed into a plastic test tube for radioactivity counting. For a blocking experiment, an excess of unlabeled DOTA-FAPI-04 was respectively co-incubated with a corresponding radiolabeled drug. After 60 minutes of incubation, the cellar uptake was terminated with a PBS solution, the cells were lysed with 1 M NaOH, the cell lysate was absorbed onto filter paper, and the filter paper was placed into a plastic test tube for radioactivity counting.

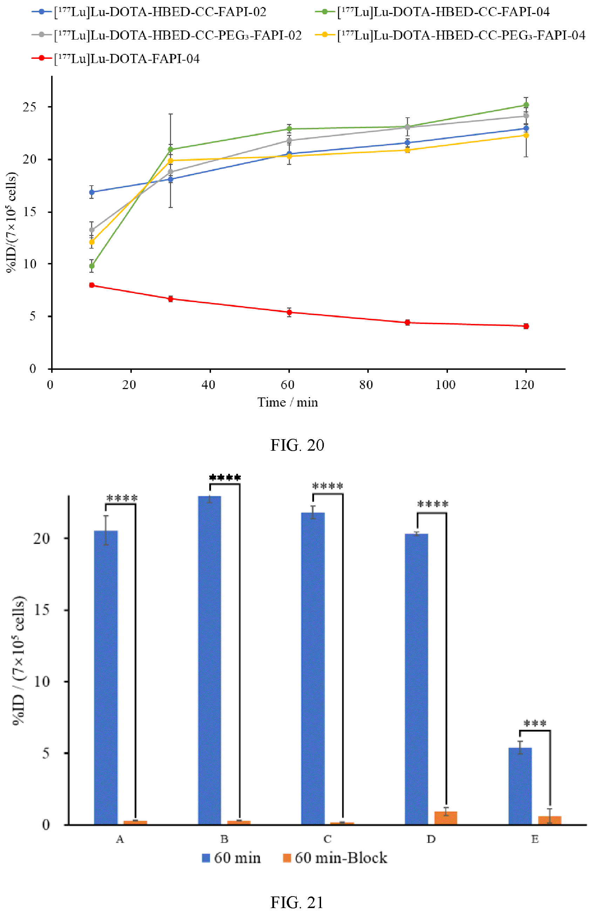

FIG. 20 shows uptake-time curves of [¹⁷⁷Lu]Lu-DOTA-HBED-CC-FAPI-02, [ ¹⁷⁷ Lu]Lu-DOTA-HBED-CC-FAPI-04, [¹⁷⁷Lu]Lu-DOTA-HBED-CC-PEG₃-FAPI-02, [¹⁷⁷Lu]Lu-DOTA-HBED-CC-PEG₃-FAPI-04 and [¹⁷⁷Lu]Lu-DOTA-FAPI-04 in HT1080-FAP cells in vitro in Application Example 2 of the present invention (n=3). FIG. 21 shows uptake and blocking results at 60 minutes of [¹⁷⁷Lu]Lu-DOTA-HBED-CC-FAPI-02, [ ¹⁷⁷ Lu]Lu-DOTA-HBED-CC-FAPI-04, [¹⁷⁷Lu]Lu-DOTA-HBED-CC-PEG₃-FAPI-02, [¹⁷⁷Lu]Lu-DOTA-HBED-CC-PEG₃-FAPI-04 and [¹⁷⁷Lu]Lu-DOTA-FAPI-04 in HT1080-FAP cells in vitro in Application Example 2 of the present invention (n=3).

### Application Example 3

Uptake of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 in HT1080-FAP cells in vitro:
HT1080-FAP cells were prepared into a cell suspension at a density of 1.25×10⁶ cells/mL, and 400 µL of the suspension was respectively seeded into a 6-well plate and cultured for 48 hours. The well plates of groups were respectively added with solutions of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 at 18.5 KBq, and incubated at 37°C. At 10, 30, 60, 90 and 120 minutes of incubation of each group, the cellar uptake was terminated with a PBS solution, the cells were lysed with 1 M NaOH, the cell lysate was absorbed onto filter paper, and the filter paper was placed into a plastic test tube for radioactivity counting. For a blocking experiment, an excess of unlabeled DOTA-FAPI-04 was respectively co-incubated with a corresponding radiolabeled drug. After 60 minutes of incubation, the cellar uptake was terminated with a PBS solution, the cells were lysed with 1 M NaOH, the cell lysate was absorbed onto filter paper, and the filter paper was placed into a plastic test tube for radioactivity counting.

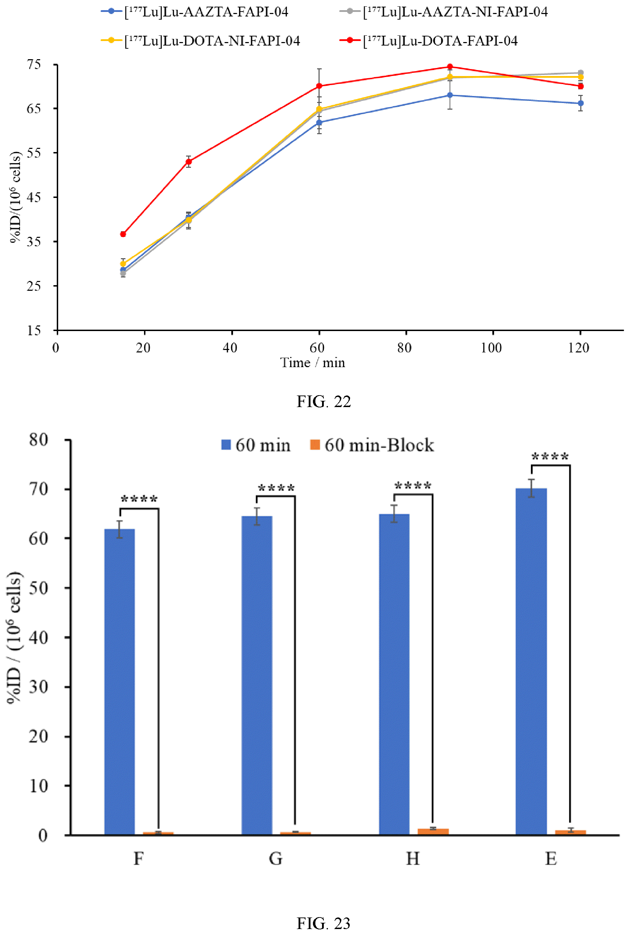

FIG. 22 shows uptake-time curves of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 in HT1080-FAP cells in vitro in Application Example 3 of the present invention (n=3). FIG. 23 shows uptake and blocking results at 60 minutes of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 in HT1080-FAP cells in vitro in Application Example 3 of the present invention (n=3).

Results of the in-vitro cellular uptake experiment indicated that the uptake of [¹⁷⁷Lu]Lu-AAZTA-FAPI-04, [¹⁷⁷Lu]Lu-AAZTA-NI-FAPI-04, [¹⁷⁷Lu]Lu-DOTA-NI-FAPI-04 and [¹⁷⁷Lu]Lu-DOTA-FAPI-04 in FAP-positive HT1080-FAP cells was gradually increased with prolonged incubation time, and the uptake of all drugs could be blocked by an excess of DOTA-FAPI-04, which indicated that all drugs specifically targeted the FAP. At all time points monitored, uptake values of [¹⁷⁷Lu]Lu-AAZTA-FAPI-04, [¹⁷⁷Lu]Lu-AAZTA-NI-FAPI-04 and [¹⁷⁷Lu]Lu-DOTA-NI-FAPI-04 were all similar to that of [¹⁷⁷Lu]Lu-DOTA-FAPI-04 or slightly higher than that of [¹⁷⁷Lu]Lu-DOTA-FAPI-04.

### Application Example 4

Uptake of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 in HT1080-FAP cells in vitro:
HT1080-FAP cells were prepared into a cell suspension at a density of 4×10⁵ cells/mL, and 500 µL of the suspension was respectively seeded into a 6-well plate and cultured for 60 hours. The well plates of groups were respectively added with solutions of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 at 222 KBq, and incubated at 37°C. At 10, 30, 60, 90 and 120 minutes of incubation of each group, the cellar uptake was terminated with a PBS solution, the cells were lysed with 1 M NaOH, the cell lysate was absorbed onto filter paper, and the filter paper was placed into a plastic test tube for radioactivity counting. For a blocking experiment, an excess of unlabeled DOTA-FAPI-04 was respectively co-incubated with a corresponding radiolabeled drug. After 60 minutes of incubation, the cellar uptake was terminated with a PBS solution, the cells were lysed with 1 M NaOH, the cell lysate was absorbed onto filter paper, and the filter paper was placed into a plastic test tube for radioactivity counting.

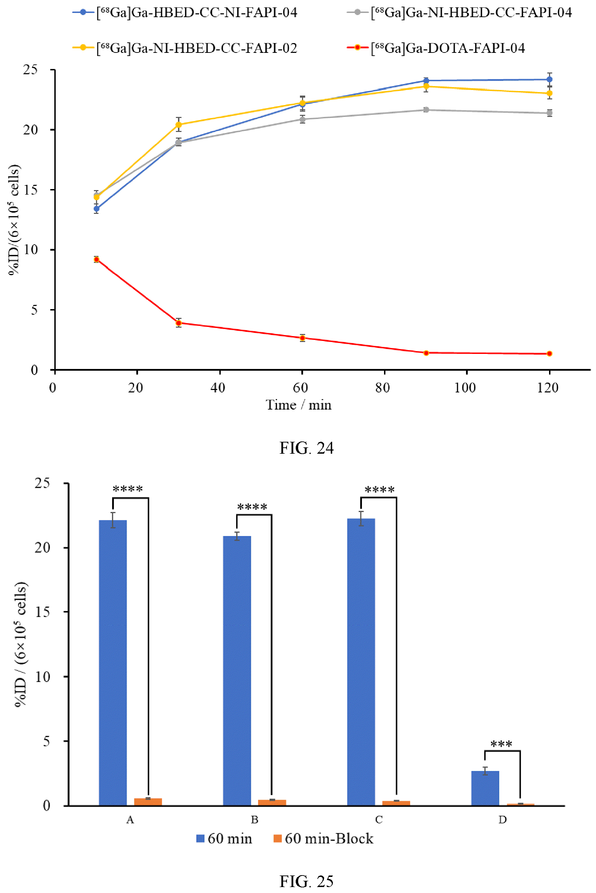

FIG. 24 shows uptake-time curves of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 in HT1080-FAP cells in vitro in Application Example 4 of the present invention (n=3). FIG. 25 shows uptake and blocking results at 60 minutes of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 in HT1080-FAP cells in vitro in Application Example 4 of the present invention (n=3).

Results of the in-vitro cellular uptake experiment indicated that the uptake of [⁶⁸Ga]Ga-HBED-CC-NI-FAPI-04, [⁶⁸Ga]Ga-NI-HBED-CC-FAPI-04 and [⁶⁸Ga]Ga-NI-HBED-CC-FAPI-02 in FAP-positive HT1080-FAP cells was gradually increased with prolonged incubation time, and the uptake of all drugs could be blocked by an excess of DOTA-FAPI-04, which indicated that all drugs specifically targeted the FAP. At all time points monitored, uptake values of [⁶⁸Ga]Ga-HBED-CC-NI-FAPI-04, [⁶⁸Ga]Ga-NI-HBED-CC-FAPI-04, [⁶⁸Ga]Ga-NI-HBED-CC-FAPI-02 and [⁶⁸Ga]Ga-DOTA-FAPI-04 were all higher than that of [⁶⁸Ga]Ga-DOTA-FAPI-04.

### Application Example 5

PET imaging of [⁶⁸Ga]Ga-AAZTA-FAPI-04, [⁶⁸Ga]Ga-AAZTA-NI-FAPI-04, [⁶⁸Ga]Ga-DOTA-NI-FAPI-04, DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in U87MG tumor-bearing mice:
the U87MG tumor-bearing mice were intravenously injected via the tail vein with [⁶⁸Ga]Ga-AAZTA-FAPI-04, [⁶⁸Ga]Ga-AAZTA-NI-FAPI-04, [⁶⁸Ga]Ga-DOTA-NI-FAPI-04, DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 or [⁶⁸Ga]Ga-DOTA-FAPI-04 (positive control) at 5.55 MBq respectively. Subsequently, under isoflurane anesthesia, small-animal PET/CT imaging was carried out at 10, 30, 60, 90, and 120 minutes after administration. For a blocking group, each drug was mixed uniformly with an excess of DOTA-FAPI-04 and then administered intravenously to the tumor-bearing mice via the tail vein, and imaging was carried out at 60 minutes respectively.

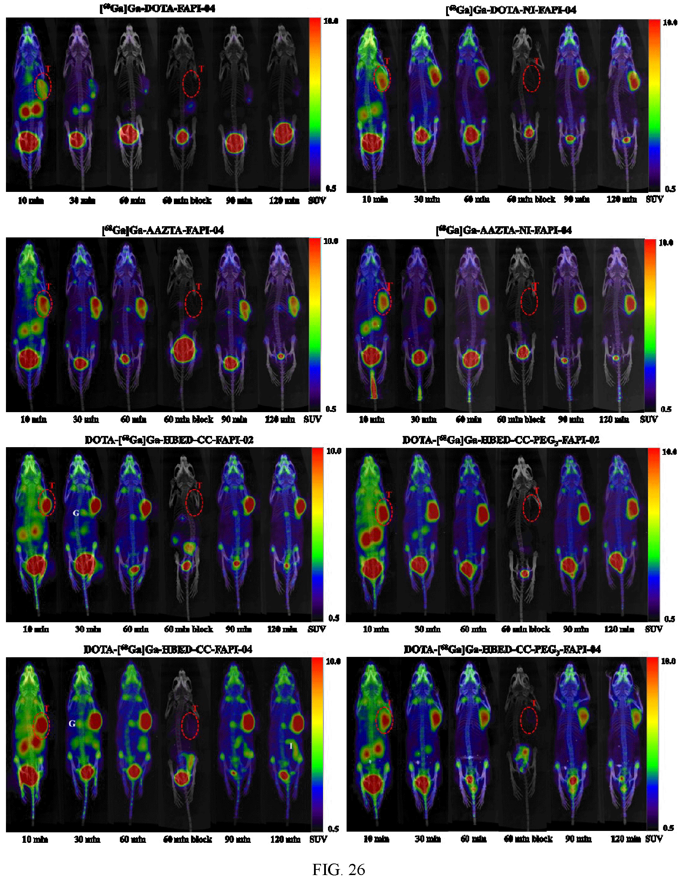

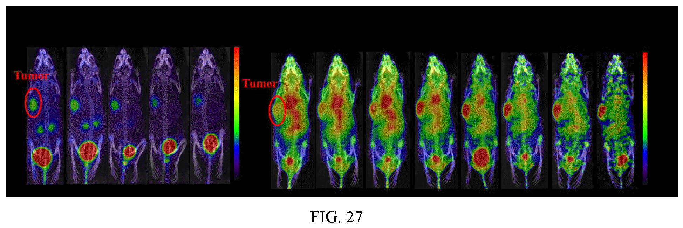

FIG. 26 shows PET/CT imaging results in U87MG tumor-bearing mice of [⁶⁸Ga]Ga-AAZTA-FAPI-04, [⁶⁸Ga]Ga-AAZTA-NI-FAPI-04, [⁶⁸Ga]Ga-DOTA-NI-FAPI-04, DOTA-[⁶⁸Ga]Ga-HBED-CC-FAPI-02, DOTA-[⁶⁸Ga]Ga-HBED-CC-FAPI-04, DOTA-[⁶⁸Ga]Ga-HBED-CC-PEG₃-FAPI-02, DOTA-[⁶⁸Ga]Ga-HBED-CC-PEG₃-FAPI-04 and [⁶⁸Ga]Ga-DOTA-FAPI-04 in Application Example 4 of the present invention.

The PET/CT imaging results in the U87MG tumor-bearing mice indicated that all compounds were taken up in tumor tissue. Furthermore, the blocking experiment indicated that the tumor uptake of the drugs could be blocked by an excess of DOTA-FAPI-04, which indicated that the drugs specifically targeted the FAP. In addition, the tumor uptake and retention of all tested drugs were higher than that of the positive control [⁶⁸Ga]Ga-DOTA-FAPI-04. Moreover, AAZTA-FAPI-04, AAZTA-NI-FAPI-04, DOTA-NI-FAPI-04, DOTA-HBED-CC-FAPI-02, DOTA-HBED-CC-FAPI-04, DOTA-HBED-CC-PEG₃-FAPI-02 and DOTA-HBED-CC-PEG₃-FAPI-04 could all be labeled with a therapeutic nuclide such as Lu-177, and were expected to become novel FAP-targeting radioactive theranostic drugs.

The above are only the preferred embodiments of the present invention, and are not intended to limit the implementation scope of the present invention, which means that all equivalent changes and modifications made according to the contents in the patent scope and the specification of the present invention shall belong to the scope of the present invention.

## Claims

1. A radiolabeled FAPI complex, **characterized in that**, the radiolabeled FAPI complex has a general structural formula as follows: 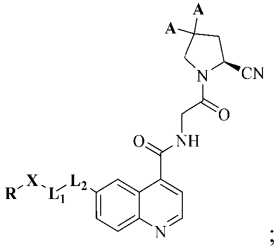
wherein, R is a nitroimidazole group or -COOH, and the nitroimidazole group is selected from any one of the following groups: 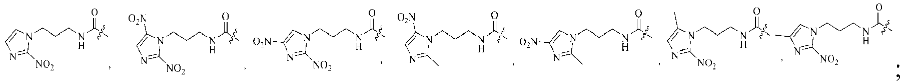
X is a chelating group or a chelating structure chelated with a radionuclide, and selected from any one of the following groups: 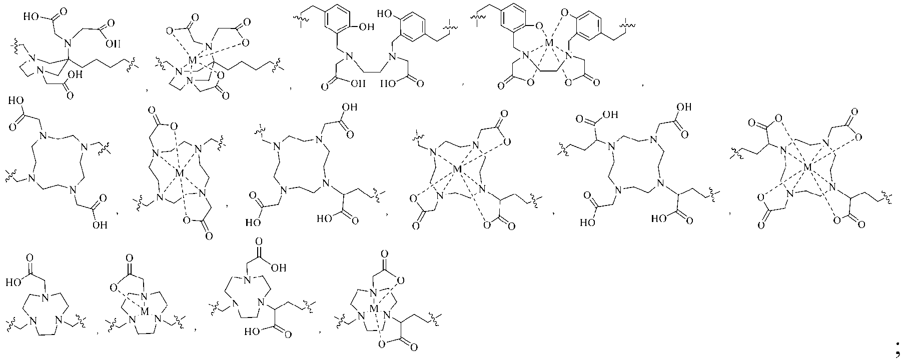
wherein, M comprises, but is not limited to, ⁶⁷Ga³⁺, ⁶⁸Ga³⁺, [Al¹⁸F]²⁺, ⁶⁴Cu²⁺, ⁶⁷Cu²⁺, ¹¹¹In³⁺, ¹⁷⁷Lu³⁺, 86Y³⁺, 90Y³⁺, ⁴⁴Sc³⁺, ⁴⁷Sc³⁺, ²²⁵Ac³⁺ , ²¹²Pb²⁺ , ²⁰³Pb²⁺ , ²¹³Bi³⁺ or ²¹²Bi³⁺;
L₁ and L₂ are linking groups, wherein the L₁ is selected from any one of the following groups: 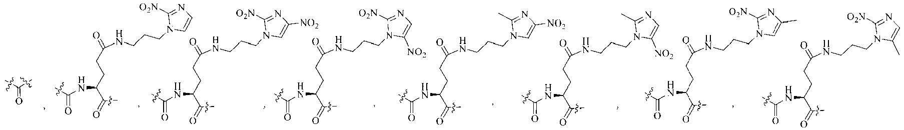 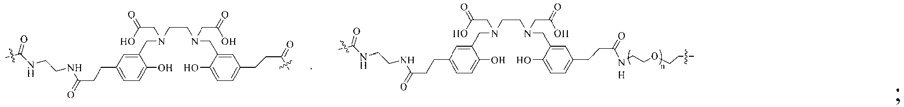
wherein, n is an integer from 0 to 6;
the L₂ is selected from any one of the following groups: 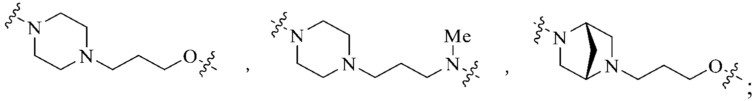
A is an adjusting atom of an FAP-targeting group, and all the A atoms are H atoms or F atoms.

2. The radiolabeled FAPI complex according to claim 1, **characterized in that**, having specific structures as follows:

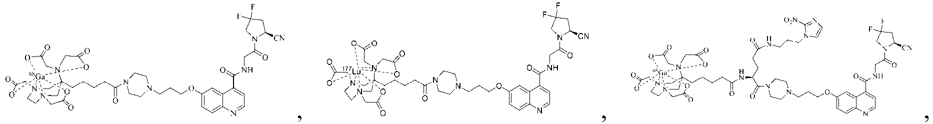

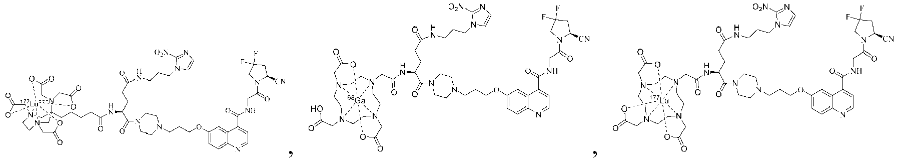

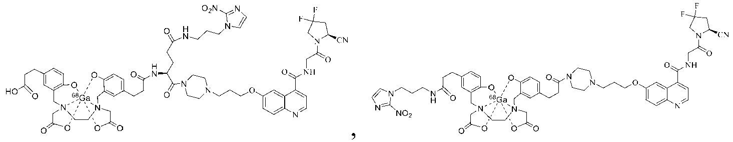

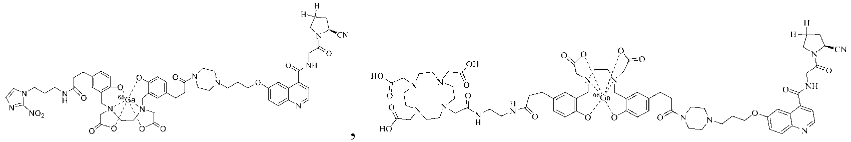

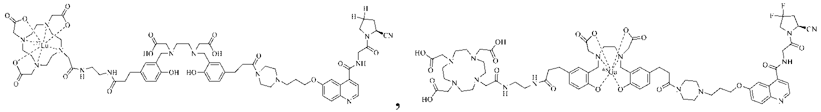

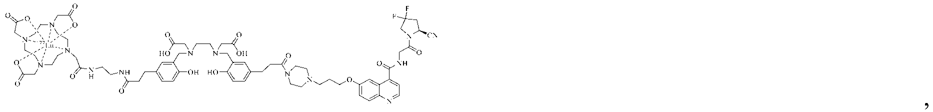

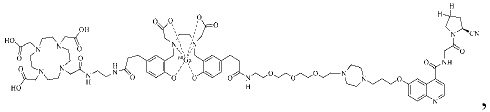

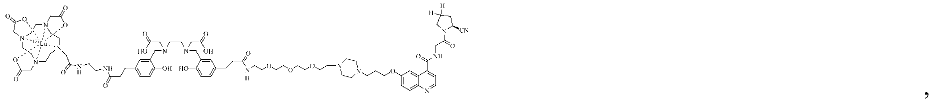

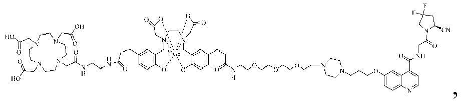

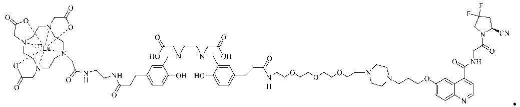

3. A preparation method for a radiolabeled FAPI complex, **characterized in that**, comprising steps as follows:
(1) dissolving nitroimidazole and anhydrous potassium carbonate in ultra-dry *N*,*N*-dimethylformamide, stirring at room temperature, and adding tert-butyl *N*-(3-bromopropyl)carbamate, and after the reaction is completed, subjecting the reaction mixture to suction filtration and extraction, drying the organic phase, and purifying to obtain a yellow-green oily compound, and deprotecting the yellow-green oily compound with trifluoroacetic acid to obtain a white solid intermediate 1;
(2) dissolving (*S*)-4-((((9H-fluoren-9-yl)methoxy)carbonyl)amino)-5-(tert-butoxy)-5-oxo-pentanoic acid in ultra-dry *N*,*N*-dimethylformamide, adding 2-(7-azabenzotriazol-1-yl)-*N,N,N',N*'-tetramethyluronium hexafluorophosphate and *N*,*N*'-diisopropylethylamine under ice bath and stirring, and adding the white solid intermediate 1 obtained in the step (1), after the reaction is completed, extracting the reaction mixture, drying the organic phase, and purifying to obtain a pale yellow solid, deprotecting the pale yellow solid with trifluoroacetic acid to obtain a yellow oily substance, dissolving the yellow oily substance in *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, adding FAPI deprotected with trifluoroacetic acid to remove a Boc group (specifically comprising (*S*)-6-[3-(4-Boc-1-piperazinyl)propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1*S*, 4*S*)-*tert*-butyl 5-(3-((4-((2-((*S*)-2-cyano-4,4-difluoropyrrolidin-1-yl)-2-oxoethyl)carbamoyl)quinolin-6-yl)oxy)propyl)-2,5-diazabicyclo[2.2.1]heptane-2-carboxylate, (*S*)-tert-butyl 4-(3-((4-((2-(2-cyano-4,4-difluoropyrrolidin-1-yl)-2-oxoethyl)carbamoyl)quino-lin-6-yl)(methyl)amino)propyl)piperazine-1-carboxylate), and after the reaction is completed, extracting the reaction mixture, drying the organic phase, and purifying to obtain a pale yellow oily substance, and deprotecting the pale yellow oily substance with diethylamine to obtain a pale yellow oily intermediate 2 (NI-FAPI);
(3) dissolving FAPI (specifically comprising (*S*)-6-[3-(4-Boc-1-piperazinyl)propoxy]-*N-*[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)-carbamoyl)quinolin-6-yl)oxy)propyl)-2,5-diazabicyclo[2.2.1]heptane-2-carboxylate, (*S*)-tert-butyl 4-(3-((4-((2-(2-cyano-4,4-difluoropyrrolidin-1-yl)-2-oxoethyl)carbamoyl)quino-lin-6-yl)(methyl)amino)propyl)piperazine-1-carboxylate) in trifluoroacetic acid, stirring at room temperature, removing the solvent by rotary evaporation to obtain a yellow oily substance, dissolving the yellow oily substance in anhydrous acetonitrile, adding anhydrous potassium carbonate, stirring at room temperature, adding a PEG chain (BocNH(CH₂CH₂O)ₙCH₂CH₂Br) under ice bath, refluxing the mixture to react overnight, subjecting the reaction mixture to suction filtration, removing the solvent by evaporation, and purifying to obtain a pale yellow oiy intermediate 3 (PEGₙ-FAPI);
(4) dissolving a chelating agent in ultra-dry *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, and adding *N*-Cbz-1,2-ethanediamine, and after the reaction is completed, extracting the reaction mixture with ethyl acetate and saturated brine, drying the organic phase with anhydrous sodium sulfate, purifying by silica gel column chromatography to obtain a yellow solid, and deprotecting the yellow solid with hydrogen to obtain a white solid substance; and dissolving 3,3'-(((2,2,13,13-tetramethyl-4,11-dioxo-3,12-dioxa-6,9-diazatetradecane-6,9-diyl)bis(methylene))bis(4-hydroxy-3,1-phenylen e))dipropanoic acid (HBED-CC(*t*Bu)₂) in ultra-dry *N*,*N*-dimethylformamide, adding 2-(7-azabenzotriazol-1-yl)-*N*,*N*,*N*',*N*'-tetramethyluronium hexafluorophosphate and N,N'-diisopropylethylamine under ice bath and stirring, and adding the white solid substance, and after the reaction is completed, extracting the reaction mixture, drying the organic phase, and purifying to obtain a colorless solid intermediate 4 (Chelator-HBED-CC(*t*Bu)₂);
(5) dissolving a chelating agent in ultra-dry *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, and adding the intermediate 2 prepared in the step (2) or FAPI deprotected with trifluoroacetic acid to remove a Boc group (specifically comprising (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((S)-2-cyano-4,4-difluoropyrrolidin-1-yl)-2-oxoethyl)-carbamoyl)quinolin-6-yl)oxy)propyl)-2,5-diazabicyclo[2.2.1]heptane-2-carboxylate and (*S*)-tert-butyl 4-(3-((4-((2-(2-cyano-4,4-difluoropyrrolidin-1-yl)-2-oxoethyl)carbamoyl)quinolin-6-yl)-(methyl)amino)propyl)piperazine-1-carboxylate); and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain a Chelator-NI-FAPI or Chelator-FAPI labeling precursor;
(6) dissolving a chelating agent in ultra-dry *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, and adding the intermediate 1 prepared in the step (1) to react at room temperature overnight, after the reaction is completed, extracting the reaction mixture, drying the organic phase, purifying to obtain a yellow oily substance, dissolving the yellow oily substance in ultra-dry *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, and adding FAPI deprotected with trifluoroacetic acid to remove a Boc group (specifically comprising (*S*)-6-[3-(4-Boc-1-piperazinyl)propoxy]-*N*-[2-(2-cyano-4,4-difluoro-1-pyrrolidinyl)-2-oxoethyl]-quinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1S,4S)-tert-butyl 5-(3-((4-((2-((*S*)-2-cyano-4,4-difluoro-pyrrolidin-1-yl)-2-oxoethyl)carbamoyl)quinolin-6-yl)oxy)propyl)-2,5-diazabicyclo[2.2.1]heptane -2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) to react at room temperature overnight, and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain an NI-Chelator-FAPI labeling precursor;
(7) dissolving the intermediate 4 prepared in the step (4) in *N*,*N*-dimethylformamide, adding 2-(7-azabenzotriazol-1-yl)-*N*,*N*,*N*',*N*'-tetramethyluronium hexafluorophosphate and *N*,*N*'-diisopropylethylamine under ice bath and stirring, and adding FAPI deprotected with trifluoroacetic acid to remove a Boc group (specifically comprising (*S*)-6-[3-(4-Boc-1-piperazinyl)propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1*S*,4*S*)-tert-butyl 5-(3-((4-((2-((*S*)-2-cyano-4,4-difluoropyrrolidin-1-yl)2-oxoethyl)carbamoyl)quinolin-6-yl)oxy)propyl)-2,5-diazabicyclo[2.2.1]heptane-2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) or the intermediate 3 prepared in the step (3), and after the reaction is completed, extracting the reaction mixture, drying the organic phase, purifying to obtain a pale yellow oily substance, deprotecting the pale yellow oily substance with trifluoroacetic acid, and purifying by semi-preparative HPLC to obtain a Chelator-HBED-CC-(PEG)ₙ-FAPI labeling precursor; and
(8) dissolving the labeling precursors obtained in the steps (5), (6) and (7) in dimethyl sulfoxide, adding a sodium acetate buffer solution and a radionuclide-containing solution, and heating to obtain a corresponding radiolabeled product.

4. The preparation method for the radiolabeled FAPI complex according to claim 3, **characterized in that**, in the step (1), an amount of the nitroimidazole added is 1 eq.; an amount of the anhydrous potassium carbonate added is 3-5 eq.; an amount of the ultra-dry *N*,*N*-dimethylformamide added is 10-20 mL; an amount of the tert-butyl *N*-(3-bromopropyl)carbamate added is 1-2 eq.; and the reaction mixture is subjected to suction filtration through celite, the filtrate is extracted with ethyl acetate and saturated brine, and the organic phase is dried with anhydrous sodium sulfate, and purified by silica gel column chromatography.

5. The preparation method for the radiolabeled FAPI complex according to claim 4, **characterized in that**, in the step (2), an amount of the (S)-4-((((9H-fluoren-9-yl)-methoxy)carbonyl)amino)-5-(tert-butoxy)-5-oxopentanoic acid is 1 eq.; an amount of the ultra-dry *N*,*N*-dimethylformamide added is 10-20 mL; an amount of the 2-(7-azabenzotriazol-1-yl)-*N*,*N*,*N*',*N*'-tetramethyluronium hexafluorophosphate and the *N*,*N*'-diisopropylethylamine added is 1-2 eq.; an amount of the yellow oily substance added is 1-1.5 eq.; the amount of the ultra-dry *N*,*N*-dimethylformamide added is 5-10 mL; the amount of the 2-(7-azabenzotriazol-1-yl)-*N*,*N*,*N*',*N*'-tetramethyluronium hexafluorophosphate and the *N*,*N*'-diisopropylethylamine added is 1.3-1.5 eq.; an amount of the FAPI deprotected with trifluoroacetic acid to remove the Boc group (specifically comprising (*S*)-6-[3-(4-Boc-1-piperazinyl)propoxy]-*N*-[2-(2-cyano-4,4-difluoro-1-pyrrolidinyl)-2-oxoethyl]-quinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1*S*,4*S*)-tert-butyl 5-(3-((4-((2-((S)-2-cyano-4,4-difluoropyrrolidin-1-yl)-2-oxoethyl)carbamoyl)quinolin-6-yl)oxy)propyl)-2,5-diazabicyclo[2.2.1 ]heptane-2-carboxylate, (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added is 1 eq.; the reaction mixture is extracted with ethyl acetate and saturated brine, and the organic phase is dried with anhydrous sodium sulfate and then purified by silica gel column chromatography; and the reaction mixture is extracted with ethyl acetate and saturated brine, and the organic phase is dried with anhydrous sodium sulfate and then purified by the flash chromatograph.

6. The preparation method for the radiolabeled FAPI complex according to claim 5, wherein, in the step (3), the mixture is refluxed to react at 80°C; the reaction mixture is subjected to suction filtration through celite to remove potassium carbonate, the solvent is removed by rotary evaporation under reduced pressure from the filtrate, and the residue is purified by the flash chromatograph; an amount of the FAPI (specifically comprising (*S*)-6-[3-(4-Boc-1-piperazinyl)propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)-carbamoyl)quinolin-6-yl)oxy)propyl)-2,5-diazabicyclo[2.2.1]heptane-2-carboxylate, (S)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added is 1 eq.; an amount of the trifluoroacetic acid added is 3-5 mL; an amount of the yellow oily substance added is 1 eq.; an amount of the anhydrous potassium carbonate added is 4-5 eq.; and the PEG chain is tert-butyl (2-(2-(2-(2-bromoethoxy)ethoxy)ethoxy)ethyl)carbamate, an amount of which is 1.2-1.5 eq.

7. The preparation method for the radiolabeled FAPI complex according to claim 6, **characterized in that**, in the step (4), the chelating agent is 5-(6-(bis(2-(tert-butoxy)-2-oxoethyl)amino)-1,4-bis(2-(tert-butoxy)-2-oxoethyl)-1,4-diazepan-6-yl)pentanoic acid (AAZTA(*t*Bu)₄), 2-(4,7,10-tris(2-(tert-butoxy)-2-oxoethyl)-1,4,7,10-tetraazacyclododecan-1-yl)-acetic acid (DOTA(*t*Bu)₃), 5-(tert-butoxy)-5-oxo-4-(4,7,10-tris(2-(tert-butoxy)-2-oxoethyl)-1,4,7,10-tetraazacyclododecan-1-yl)pentanoic acid (DOTAGA(*t*Bu)₄), 4-(4,10-bis(2-(tert-butoxy)-2-oxoethyl)-7-(1,5-di(tert-butoxy)-1,5-dioxopentan-2-yl)-1,4,7,10-tetraazacyclodod ecan-1-yl)-5-(tert-butoxy)-5-oxopentanoic acid (DOTA(GA)₂ (*t*Bu)₃), 2-(4,7-bis(2-(tert-butoxy)-2-oxoethyl)-1,4,7-triazacyclopentan-1-yl)acetic acid (NOTA(*t*Bu)₂) or 4-(4,7-bis(2-(tert-butoxy)-2-oxoethyl)-1,4,7-triazolan-1-yl)-5-(tert-butoxy)-5-oxopentanoic acid (NODAGA(*t*Bu)₃); an amount of the chelating agent added is 1 eq.; an amount of the ultra-dry *N*,*N*-dimethylformamide added is 15-20 mL; an amount of the 2-(7-azabenzotriazol-1-yl)-*N*,*N*,*N*',*N*'-tetramethyluronium hexafluorophosphate added is 1 eq.; an amount of the *N*,*N*'-diisopropylethylamine added is 3 eq.; an amount of the *N*-Cbz-1,2-ethanediamine added is 1.2-1.5 eq.; the reaction mixture is extracted with ethyl acetate and saturated brine, and the organic phase is dried with anhydrous sodium sulfate, and purified by silica gel column chromatography to obtain the yellow solid; an amount of the HBED-CC(*t*Bu)₂ added is 1 eq.; an amount of the ultra-dry *N*,*N*-dimethylformamide added is 10-20 mL; an amount of the 2-(7-azabenzotriazol-1-yl)-*N*,*N*,*N*',*N*'-tetramethyluronium hexafluorophosphate added is 1 eq.; an amount of the *N*,*N*'-diisopropylethylamine added is 3 eq.; and the white solid substance is added, and after the reaction is completed, the reaction mixture is extracted with ethyl acetate and saturated brine, and the organic phase is dried with anhydrous sodium sulfate, and purified by the flash chromatograph to obtain the colorless solid intermediate 4 (Chelator-HBED-CC(*t*Bu)₂).

8. The preparation method for the radiolabeled FAPI complex according to claim 7, **characterized in that**, in the step (5), the chelating agent is 5-(6-(bis(2-(tert-butoxy)-2-oxoethyl)amino)-1,4-bis(2-(tert-butoxy)-2-oxoethyl)-1,4-diazepan-6-yl)pentanoic acid (AAZTA(*t*Bu)₄), 2-(4,7,10-tris(2-(tert-butoxy)-2-oxoethyl)-1,4,7,10-tetraazacyclododecan-1-yl)-acetic acid (DOTA(*t*Bu)₃), 3,3'-(((2,2,13,13-tetramethyl-4,11-dioxo-3,12-dioxa-6,9-diazatetra-decane-6,9-diyl)bis(methylene))bis(4-hydroxy-3,1-phenylene))dipropanoic acid (HBED-CC(*t*Bu)₂), 5-(tert-butoxy)-5-oxo-4-(4,7,10-tris(2-(tert-butoxy)-2-oxoethyl)-1,4,7,10-tetraazacyclododecan-1-yl)pentanoic acid (DOTAGA(*t*Bu)₄), 4-(4,10-bis(2-(tert-butoxy)-2-oxoethyl)-7-(1,5-di(tert-butoxy)-1,5-dioxopentan-2-yl)-1,4,7,10-tetraazacyclododecan-1-yl)-5-(tert-butoxy)-5-oxopentanoic acid (DOTA(GA)₂ (*t*Bu)₅), 2-(4,7-bis(2-(tert-butoxy)-2-oxoethyl)-1,4,7-triazacyclopentan-1-yl)acetic acid (NOTA(*t*Bu)₂) or 4-(4,7-bis(2-(tert-butoxy)-2-oxoethyl)-1,4,7-triazolan-1-yl)-5-(tert-butoxy)-5-oxopentanoic acid (NODAGA(*t*Bu)₃); an amount of the chelating agent added is 1-1.2 eq.; an amount of the ultra-dry *N*,*N*-dimethylformamide added is 2-5 mL; an amount of the 2-(7-azabenzotriazol-1-yl)-*N*,*N*,*N*',*N*'-tetramethyluronium hexafluorophosphate added is 1-1.5 eq.; an amount of the *N*,*N*'-diisopropylethylamine added is 1.5-3 eq.; an amount of the intermediate 2 or the FAPI deprotected with trifluoroacetic acid to remove the Boc group (specifically comprising (*S*)-6-[3-(4-Boc-1-piperazinyl)propoxy]-*N*-[2-(2-cyano-4,4-difluoro-1pyrrolidinyl)-2-oxoethyl]quinoline-4-carboxamide, (*S*)-6-[3-(4-Boc-1-piperazinyl)propoxy]-*N*-[2-(2-cyano-pyrrolidinyl)-2-oxoethyl]quinoline-4-carboxamide, (1*S*,4*S*)-tert-butyl 5-(3-((4-((2-((S)-2-cyano-4,4-difluoropyrrolidin-1-yl)-2-oxoethyl)carbamoyl)quinolin-6-yl)oxy)propyl)-2,5-diaza bicyclo[2.2.1]heptane-2-carboxylate and (*S*)-tert-butyl 4-(3-((4-((2-(2-cyano4,4-difluoro-pyrrolidin-1-yl)-2-oxoethyl)carbamoyl)quinolin-6-yl)(methyl)amino)propyl)piperazine-1-carbox ylate) added is 1 eq.; and after the reaction is completed, the reaction mixture is extracted with ethyl acetate and saturated brine, and the organic phase is dried with anhydrous sodium sulfate, and purified by the flash chromatograph.

9. The preparation method for the radiolabeled FAPI complex according to claim 8, in the step (6), the chelating agent is 3,3'-(((2,2,13,13-tetramethyl-4,11-dioxo-3,12-dioxa-6,9-diaza-tetradecane-6,9-diyl)bis(methylene))bis(4-hydroxy-3,1-phenylene))dipropanoic acid (HBED-CC(*t*Bu)₂) or 4,4'-(4,10-bis(2-(tert-butoxy)-2-oxoethyl)-1,4,7,10-tetraazacyclo-dodecane-1,7-diyl)bis(5-(tert-butoxy)-5-oxopentanoic acid) (DOTA(GA)₂(*t*Bu)₄); an amount of the chelating agent added is 1 eq.; an amount of the ultra-dry *N*,*N*-dimethylformamide added is 2-10 mL; an amount of the 2-(7-azabenzotriazol-1-yl)-*N*,*N*,*N*',*N*'-tetramethyluronium hexafluorophosphate added is 1 eq.; an amount of the N,N'-diisopropylethylamine added is 3-5 eq.; an amount of the intermediate 1 added is 1 eq.; after the reaction is completed, the reaction mixture is extracted with ethyl acetate and saturated brine, and the organic phase is dried with anhydrous sodium sulfate, and purified by the flash chromatograph; amounts of the pale yellow oily substance, the 2-(7-azabenzotriazol-1-yl)-*N*,*N*,*N*',*N*'-tetramethyluronium hexafluorophosphate, the *N*,*N*'-diisopropylethylamine and the FAPI deprotected with trifluoroacetic acid to remove the Boc group (specifically comprising (*S*)-6-[3-(4-Boc-1-piperazinyl)-propoxy]-*N*-[2-(2-cyano-4,4-difluoro-1-pyrrolidinyl)-2-oxoethyl]quinoline-4-carboxamide, (*S*)-6-[3-(4-Boc-1-piperazinyl)propoxy]-*N*-[2-(2-cyanopyrrolidinyl)-2-oxoethyl]quinoline-4-carb oxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)propyl)-2,5-diazabicyclo[2.2.1]heptane-2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added are all 1 eq.; an amount of the ultra-dry *N*,*N*-dimethylformamide added is 7-10 mL; after the reaction is completed, the reaction mixture is extracted with ethyl acetate and saturated brine, and the organic phase is dried with anhydrous sodium sulfate, and purified by the flash chromatograph; in the step (7), an amount of the intermediate 4 added is 1 eq.; an amount of the 2-(7-azabenzotriazol-1-yl)-*N*,*N*,*N*',*N*'-tetramethyluronium hexafluorophosphate added is 1-1.5 eq.; an amount of the N,N'-diisopropylethylamine added is 4-5 eq.; an amount of the intermediate 3 or the FAPI deprotected with trifluoroacetic acid to remove the Boc group (specifically comprising (*S*)-6-[3-(4-Boc-1-piperazinyl)propoxy]-*N*-[2-(2-cyano-4,4-difluoro-1-pyrrolidinyl)-2-oxoethyl]q uinoline-4-carboxamide, (*S*)-6-[3-(4-Boc-1-piperazinyl)propoxy]-*N*-[2-(2-cyanopyrrolidinyl)-2-oxoethyl]quinoline-4-carboxamide, (1*S*,4*S*)-tert-butyl 5-(3-((4-((2-((*S*)-2-cyano-4,4-difluoropyrrolidin-1-yl)-2-oxoethyl)carbamoyl)quinolin-6-yl)oxy)propyl)-2,5-diazabicyclo[2.2.1]heptane -2-carboxylate and (*S*)-tert-butyl 4-(3-((4-((2-(2-cyano-4,4-difluoropyrrolidin-1-yl)-2-oxoethyl)carbamoyl)quinolin-6-yl)(methyl)amino)propyl)piperazine-1-carboxylate) added is 1-1.5 eq.; in the step (7), after the reaction is completed, the reaction mixture is extracted with ethyl acetate and saturated brine, and the organic phase is dried with anhydrous sodium sulfate, and purified by the flash chromatograph; and in the step (8), the radionuclide-containing solution is [⁶⁸Ga]GaCl₃, [¹⁷⁷Lu]LuCl₃ or [¹⁸F]AlF.

10. Use of the radiolabeled FAPI complex according to any one of claims 1 to 2 in manufacture of a targeted radioactive theranostic drug.
